# EUROPEAN PATENT APPLICATION

(11) **EP 2 027 856 A1**
(43) Date of publication of application: **25.02.2009**
(21) Application number: 08168609.9
(22) Date of filing: 28.06.2004
(51) Int. Cl.: A61K 31/454, A61K 31/4166, A61K 31/5377, A61P 25/18, A61P 25/22, A61P 25/24

(54) **Method of treating or preventing central nervous system disorders with compounds having selectivity benzodiazepine receptor**

(30) Priority: 11.07.2003 US 486678 P
(62) Divisional of application: 04740387.8
(71) Applicant: Boehringer Ingelheim Vetmedica GmbH, 55216 Ingelheim am Rhein (DE)
(72) Inventor: Langen, Barbara, 14169 Berlin (DE); Rundfeldt, Chris, 01640 Coswig (DE); Dost, Rita, 01217 Dresden (DE); Lüddens, Hartmund, 55129 Mainz (DE); Rabe, Holger, 55218 Ingelheim (DE)
(74) Representative: Hammann, Heinz

(57) **Abstract**

The present invention relates to the use of 1-ar(alk)ylimidazolin-2-ones which contain a disubstituted amine radical in the 4-position for the treatment or prevention of central nervous system disorders including depression, anxiety, movement disorders, and especially dystonia, and psychotic disorders, and especially schizophrenia and psychotic symptoms associated to other mental disorders.

## Description

### Field of the invention

The present invention relates to the use of 1-ar(alk)ylimidazolin-2-ones, which contain a disubstituted amine radical in the 4-position, for the treatment or prevention of central nervous system disorders, including psychotic disorders and especially schizophrenia, and depression, anxiety and dystonia and to the use of these and other agonistic substances which are subtype selective ligands for benzodiazepin receptors containing an alpha 3 subunit.

### Background of the invention

Central nervous system disorders are severe mental disorders which extremely impair daily life. For example, schizophrenia affects about 1 % of the world-wide population (Capuano et al., 2002) and mostly starts before 30 years of age implying a life-long treatment (Benes, 1993).

Psychosis, especially schizophrenia, has a heterogeneous symptomatic picture (American Psychiatric Association, 1994). The symptoms may be divided into two fractions, in the acute phase, schizophrenia is predominated by hallucinations (sensory perception in the absence of an outside stimulus) and delusions (false, fixed and unusual beliefs), for instance persecution mania. The patient is extremely agitated and loses contact to reality. These are called the positive symptoms (Davidson and Neale, 1988 ; Bailer et al., 1999). When the agitated phase abates the so called negative symptoms become obvious. The symptoms include cognitive deficits, reduced vigilance, deficits in verbal learning and memory, verbal fluency, motor function, working memory but also indifference and listlessness. Patient are unsure and anxious (Davidson and Neale, 1988; Bailer et al., 1999).

Although several antipsychotic drugs are available, the present therapy of psychosis is not satisfactory. The classic antipsychotics with a high affinity to dopamine D2 receptor show extreme movement disorders and sedative side effects (Nyberg et al., 2002). The most famous antetype of the classic antipsychotics and still a drug for first line treatment is haloperidol (Capuano et al., 2002). Because of its negative side effects and the fact that it may reduce the positive acute symptoms but not the negative symptoms of schizophrenia it does not enable the patient to return to everyday life.

This fact led to the development of novel antipsychotics, the so called atypical antipsychotics. They show a lower dopamine D2 receptor affinity and a more diverse receptor affinity profile focusing on the serotonin receptor subtype 5-HT2 (Sawa and Snyder, 2002). The change in the receptor affinity profile of the drugs reduced the movement disorder side effect but shifted the side effects to issues like extreme body weight gain, sexual disturbance, cognitive dysfunction and anhedonia. Clozapine which has emerged as a benchmark therapeutic ameliorating both positive and negative symptoms of schizophrenia and devoid of movement disorder side-effects shows agranulocytosis as a major, potentially lethal side-effect (Capuano et al., 2002). Above all, there is still a high amount of therapy resistant cases (Lindenmayer et al., 2002).

The cause of schizophrenia is not fully elucidated nor is the mechanism of action of antipsychotic drugs, especially of atypical antipsychotic drugs. There seems to be a polygenic mode of inheritance but it is also governed by non-genetic factors (Prasad et al., 2002). Growing epidemiological, genetic, and clinical neurobiological evidence indicates that abnormalities in brain development play determining roles in the pathophysiology of schizophrenia (Arnold, 1999).

The main hypothesis of schizophrenia emanates from a dysfunction of the dopaminergic system. Thus, acute psychotic symptoms may be stimulated by dopaminergic drugs (Capuano et al., 2002) and, as described above, the classical antipsychotics, like haloperidol, have a high affinity to the dopamine D2 receptor (Nyberg et al., 2002). However, the mode of action of the atypical antipsychotics indicate that there are further neurotransmitter systems involved in the development of schizophrenia. Clozapine, the benchmark antipsychotic drug and the only drug that ameliorates positive and negative symptoms of schizophrenia does not show a high affinity to dopamine D2 receptors (Gerlach, 2002). For instance, another neuro-transmitter involved in the pathophysiology of schizophrenia is serotonin (Sawa and Snyder, 2002).

The glutamatergic neurotransmitter system seems also to be involved in the development of schizophrenia. Thus, NMDA antagonists like phencyclidine and ketamine are able to stimulate schizophrenic symptoms in humans and rodents (Abi-Saab et al., 1998; Lahti et al., 2001). The animal models of psychosis based on NMDA antagonists have the advantage over dopaminergic models that they not only mimic the agitated and impulsive behaviour of the positive phase of schizophrenia but also the negative symptoms of schizophrenia like cognitive deficits. (Abi-Saab et al., 1998 ; Jentsch and Roth, 1999). Thus, this model can be used to identify new drugs with antipsychotic potential.

While the cause of most central nervous system disorders is far from understood, the neurotransmitter serotonin (5-HT) was found to play an important role in many central nervous system disorders. Although complex emotional states such as depression and anxiety, cannot solely be reduced to imbalances induced by a single neurotransmitter, deficits in the 5-HT neurotransmitter system are generally acknowledged to be prominently involved in the development of depression and anxiety (Graeff et al., 1996).

Thus, independent of the variable clinical forms of depression, such as major depressive disorder and episodes, manic, mixed and hypomanic mood episodes, depressive episodes with atypical, catatonic or melancholic features, depressive episodes with postpartum onset premenstrual dysphoric disorder, minor depressive disorder, post traumatic and acute stress disorder, depressive patients show a significant reduction of 5-HT in the cerebrospinal fluid and additional alterations in their central 5-HT system (Owens and Nemeroff, 1994). Although the underlying mechanism of depression is certainty more complex than simply a reduction in levels of 5-HT or a diminished function in this system (Delgado and Moreno, 1999) the participation of the serotonerg system in depression is most clearly shown by the therapeutic efficiency of drugs increasing the extracellular 5-HT concentration in the brain, such as selective serotonin reuptake inhibitors (SSRI). Thus, the SSRI, like fluoxetine or citalopram are described to be effective in various subgroups of depression including major depressive disorder, obsessive-compulsive disorder and patients with eating disorders (Stokes and Holtz, 1997). It is important to note that SSRIs were considered a considerable progress in the treatment of central nervous system disorders compared to non-selective monoamine uptake inhibitors which simultaneously elevate the levels of serotonine, dopamine and noradrenaline to varying extents and show accordingly more side effects.

Additionally, a long lasting increase of the extracellular 5-HT concentration in brain by, for instance SSRIs, may reduce anxiety in animals and humans (Jones et al., 2002; Stokes and Holtz, 1997). Thus, fluoxetine, a SSRI, is therapeutical used for different anxiety disorders (Nutt et al., 1999) indicating the additional modulating effect of enhanced 5-HT levels on panic disorder, agoraphobia, specific phobia, social phobia and generalized anxiety disorder. It is to make clear, that the pharmacological effect is mediated by the increased levels of the neurotransmitters resulting in an increased activation of the respective receptor and not by the specific block of the uptake transporter.

However, the late onset of the anxiolytic and antidepressive effect of compounds increasing the 5-HT level in the brain, like SSRIs, are a limiting factor of the therapeutic benefit of these drugs (Nutt et al., 1999). A human being strongly at risk to commit suicide cannot wait three weeks until the therapeutic drug will display its antidepressive, antipsychotic and/or anxiolytic effect.

In contrast to the SSRI, benzodiazepines show a rapid onset of their anxiolytic activity (Costa and Guidotti, 1996). However, the range of their therapeutic use is restricted to a relatively short period of time since the development of tolerance to the effect of the benzodiazepines and the risk of drug addiction limits their chronic use (Costa and Guidotti, 1996).

Thus, a therapeutic drug combining both mechanisms, the rapid onset of the activity of the benzodiazepines and the chronic efficiency of the SSRI, would be a great progress in the therapy of anxiety disorders as well as depression.

Another embodiment of a central nervous system disorder is dystonia. Dystonia is a movement disorder based on a malfunction of the central nervous system to control motor activity; it was previously also known as psychovegetative syndrome. It is characterised by involuntary, repetitive movements and abnormal postures partly combined by a painful cramping of the muscles (Green, 1992; Friedman and Standaert, 2001; Hamann and Richter, 2002). Depending on the subtype of dystonia the symptoms reach from focal to generalised dystonic attacks. There are atso progressive forms beginning with focal attacks during childhood. People of all ages may be affected. In Germany, there are about 80000 individuals with dystonic attacks (DDG eV, 2002).

On the basis of the distribution of symptoms dystonia may be classified in several subtypes: focal dystonias, multiple-focal or segmental dystonias, torsion dystonia, hemispheric, generalised and tardive dystonias. Focal dystonias include cervical dystonia (torticolli), blepharospasm (cramp of the eyelid), appendicular dystonia (cramp in the extremities, like the writer's cramp), oromandibular dystonia and spasmodic dysphonia (cramp of the vocal cord) (DDG eV, 2002; Friedman and Standaert, 2001).

At present, except of a few rare forms the treatment of dystonia focuses on symptomatic therapies. Focal dystonias can be treated quite successfully by botulinum toxin (Hsiung GY et al., 2002). Botulinum toxin is administered locally into the affected area and causes a relaxation of the muscle for several weeks. The treatment has to be repeated regularly. The weak point of the therapy is that some patients develop a resistance to the toxin due to antibodies raised against it and that it cannot be used when greater areas of the body are affected (Dressier et al., 2002; Hsiung et al., 2002).

The systemic pharmacotherapies of segmental and generalised dystonias are unsatisfactory. It involves anticholinergic drugs and baclofen, a pre-synaptic acting GABAB agonist, reported to favourably influence dystonia symptoms (Fahn, 1987; Green, 1992; Rawicki, 1999). The effect of anticonvulsant drugs on dystonia is inconsistent: phenobarbital and lamotrigine seems to have a pro-dystonic effect whereas gabapentin is assumed to be antidystonic (Richter and Löscher, 1999; Richter and Löscher, 2000; Siep et al., 2002).

A surgical therapy, the deep brain stimulation of the globus pallidus, for severe dystonia is still in the very first stage of development and only successful in certain types of dystonia. Most times an additional, systemic pharmacotherapy is necessary (Krack and Vercueil, 2001; Vercueil et al., 2002; Klein and Ozelius, 2002).

The mechanism of dystonia is not fully elucidated. There are many hints to a dysfunction of the basal ganglia (Gernert et al., 2002 ; Herrero et al., 2002). The mechanism coordinating the information of somatosensory afferences and gating them to the motor system is supposed to be impaired (Herrero et al., 2002). Dystonia may be caused by brain trauma or a stroke but about 80 % of generalised dystonias are idiopathic and seem to be inheritable with a different degree of penetrance (Pauls and Korczyn, 1990). Currently 13 different forms of dystonia can be distinguished on a genetic basis (dystonia types 1-13) (Klein and Ozelius, 2002). A gene mutation has been identified for three, rare subtypes of generalised dystonias, for instance the L-dopa responsive type (Thyagarajan; 2001).

The genetic hamster model of paroxysmal dystonia is one of the few clearly defined animal models of dystonia (Hamann and Richter, 2002).

Anxiety disorders are highly prevalent with an increasing incidence world-wide. Benzodiazepines are still regarded as the most effective drugs for the treatment of anxiety disorders showing a fast onset of the anxiolytic activity. However, they also show undesired side-effects, such as ataxia, sedation, skeletal muscle relaxation, amnesia ethanol and barbiturate interaction. Major problems are also the development of tolerance to their therapeutic effects and the potential for drug abuse (Costa and Guidotti, 1996; Atack, 2003).

As mentioned above, class of drugs used for treatment of anxiety are the selective serotonine re-uptake inhibitors (SSRIs). These drugs are well established as antidepressants and do not induce major side-effects of benzodiazepines, such as tolerance or drug abuse, but the late onset of their anxiolytic and antidepressive effect are a limiting factor of their therapeutic benefit (Nutt et al., 1999). Besides, their therapeutic use is affected by weight gain and sexual dysfunctions which leads patients to discontinue the therapy (Perna et al., 2001). A combination of the positive effects of both, benzodiazepine receptor ligands and SSRIs, could serve as template for an ideal anxioiytic. In conclusion, there still is a high need for an ideal anxiotytic.

One attempt to reduce these major side-effects of drugs binding to the benzodiazepine recognition site at the GABA_{A} receptor, namely sedation, would be to develop drugs being highly selective to certain GABA_{A} receptor subtypes (Costa and Guidotti, 1996). During the last years pharmacological and genetical studies revealed different α-subunits of receptors being responsible for different behavioural symptoms induced by benzodiazepines (Atack, 2003).

Thus, alpha1-subunits containing receptors are described to mediate the sedative and anticonvulsant effect of the benzodiazepines (Costa and Guidotti, 1996; Crestani et al., 2000; Dubinsky et al., 2002). GABA_{A} receptors comprising alpha5-subunits should play a part in amnesia induced by benzodiazepines while alpha2-and alpha3-subunits containing receptors were seen to be responsible for the anxiolytic activity of these compound class (Costa and Guidotti, 1996; Low et al., 2000; Dubinsky et al., 2002). However, the subtype specific effects of the benzodiazepines are not yet fully understood and there is an ongoing controversal discussion on the role of different subunits which is further complicated by the fact that in addition to different alpha subunits also different beta and gamma subunits as well as additional subunits are reported and contribute to heterogeneity. Some findings indicate that the sedative effect of the benzodiazepines, for instance, is not exclusively mediated by the alpha1-subunit containing receptor subtype but by additional mechanism and that alpha1-subunits solely play a predominant role in ataxia (Platt et al., 2002). Tauber et al. (2003) found that the loss of righting reflex due to the simultaneous administration of diazepam and ethanol is not seen in alpha2-subunit-mutant mice (combined with a loss of receptor activity) but the drop of locomotor activity (sign of sedation) caused by the administration of both compound was found in alpha1-, alpha2-, alpha3-and alpha5-subunit-mutant mice. This lack of knowledge of the function of different benzodiazepine receptor subtypes which are heterogenously expressed in the brain is mainly caused by a lack of highly subtype selective ligands for different subtypes.

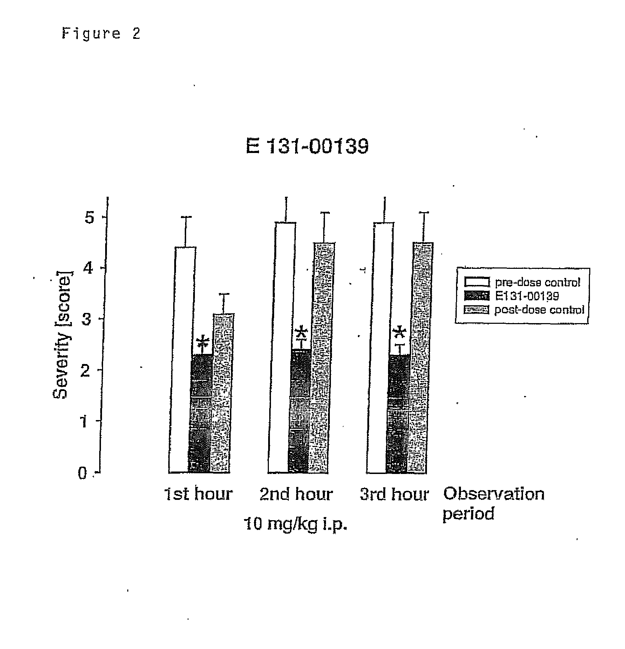

Nevertheless, based on the above mentioned findings made on the basis of genetically modified mice with compromized alpha subunits no longer capable of mediating the benzodiazepine effect, several companies have initiated research programs to develop benzodiazepine receptor ligands selective for both the alpha 2 and alpha 3 subunit with reduced activity on the alpha 1 subunit. (Low et al., 2000; Griebel et al., 2001). Few compounds of that kind have been described and the degree of subtype selectivity seems to be rather limited. The functional selectivity is achieved by different degrees of partial agonism on individual receptors. For example, the substance SL651498 was shown to act with approximately 40-50% partial agonistic activity on alpha 1 and alpha 5 containing receptors while acting with 100% partial agonistic activity on alpha 2 receptors and 70% activity on alpha 3 receptors. This may result indeed in functional subtype selectivity, however only with regard to pharmacological effects which require a high degree of activation of the receptors. For effects requiring less than 50% activation such compounds will act like non-selective agonists as can be seen in figure 2 in the paper by Griebel et al., 2001. The discussion is still out what degree of receptor activation is needed for which effect, but even partial non-selective agonists such as imidazenil have been found to be sedative in patients (Attack, 2003). It thus remains questionable whether the strategy of functional partial agonism can deliver the intended selectivity for de-selecting the alpha 1 subunit. Despite this fact, others are using the same approach (Dawson, 1998 ; McKernan, 1998). NS2710 and an undisclosed new compound in clinical development derived from the research and development program of Merck (Adis Data Information, 2003; Goodacre et al., 2002; Chambers et al., 2001) did not yet deliver the anticipated clinical profile and proved to be sedative and not free of addictive potential (Atack, 2003). Taken these results together, no major progress has been made to date developing new benzodiazepine receptor ligands, especially with high subtype selectivity. Furthermore, the knowledge on the role of different subunits in physiology and pathophysiology of diseases is still very limited, again due to a lack of selective compounds.

While knowledge of the role of the subunits alpha 1 and alpha 2 was the target of at least some work, even less is known on the alpha 3 subunit, which is a subunit with rather selective distribution in the brain. To date no ligands with any selectivity for the alpha 3 subunit are available, all above mentioned drugs were designed to be and are at best somewhat selective for both the alpha 2 in combination with the alpha 3 subunit, de-selecting to a certain degree the alpha 1 subunit. There are only few studies about the alpha3-subunit containing receptor. In an immunocytochemical study the alpha3-subunit is described to be abundantly expressed in the cholinergic neurons of the striatum, septum and pedunculo-pontine nucleus and the dopaminergic neurons of the substantia nigra pars compacta whereas these cells express only few alpha2-subunits (Rodriguez-Pallares et al., 2001). On the dopaminergic neurons of the pars compacta of the substancia nigra alpha3 subunits mainly seem to be accompanied by alpha 4 subunits (the alpha 4 subunit is not sensitive to benzodiazepines) whereas mRNAs of alpha2-subunits were not found (Guyon et al., 1999). Noradrenergic neurons in the locus coeruleus are described to be immunoreactive for the alpha3-and the alpha2-subunit. All these brain areas were immunonegative for alpha1-subunits (Rodriguez-Pallares et al., 2001).

Similarily, the serotonergic neurons in the raphe show a high level of labelling of alpha3-subunits, while there are only few neurons expressing the alpha2 subunit (Rodriguez-Pallares et al., 2001). Additionally, Gao et al. (1993) found that the vast majority of serotonergic neurons in the raphe express strong alpha3-subunitimmunoreactivity, but are devoid of alphal-subunit staining whereas both subunits are present in GABAerg neurons of the raphe. However, all these studies were labelling studies and do not permit any clear view on the pharmacology mediated via the alpha 3 subunit.

When locally administering muscimol, a GABA_{A} agonist, and bicuculline, a GABA_{A} antagonist, in the dorsal and medial raphe an effect on local and nucleus accumbens extracellular 5-HT levels has been found in a microdialysis study (Tao and Auerbach, 2000). This indicates that the alpha3-subunit containing GABA_{A} receptors may be involved in serotonergic neurotransmission.

### Description of the invention

From the above, it becomes clear that the current methods and possibilities of treating central nervous system disorders including psychotic disorders, depression, anxiety and movement disorders like dystonia are insufficient and at least partly show severe side effects.

Therefore, it was an object of the present invention to provide further possibilities of treating or preventing such central nervous system disorders in mammals and especially to present a treatment for human use.

According to a first subject of the present invention, this object was solved by a method of treating or preventing central nervous system disorders including psychotic disorders, depression, anxiety and movement disorders and/or psychotic symptoms associated to other mental disorders by administering an effective amount of at least one 1-ar(alk)ylimidazolin-2-one of formula (I) 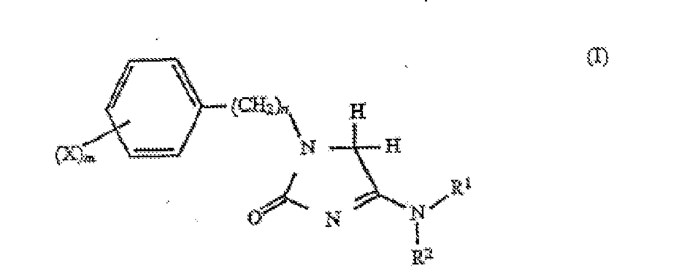 in which X is hydrogen, a C₁₋₄-alkyl, C₁₋₄ alkoxy, trifluoromethyl, or a halogen residue, R¹ and R² are independently of each other a C₁₋₄-alkyl, C₃₋₁₀ cycloalkyl or C₃₋₁₀ heteroalkyl residue, or R¹ and R² are together a C₂₋₆ akylene residue in which a -CH₂-group is optionally replaced by oxygen, nitrogen or sulfur, n is 0 or 1, and m is 0 or a cardinal number from 1 to 5 to a patient in need thereof.

According to the present invention, it has been found that surprisingly the compounds of formula I are highly efficient in treating the following central nervous system disorders:

### 1. Psychosis and psychotic episodes

Different types of schizophrenia (for instance paranoid, disorganized, catatonic undifferentiated or residual) and bipolar mood disorders, such as manic depression and the post-psychotic depressive disorders of schizophrenia. Psychotic episodes to be seen with schizophreniform disorders, schizoaffective disorders, delusional disorders, substance-induced psychotic disorders (for instance induced by alcohol, amphetamine, cannabis, cocaine, halluzinogens, inhalants, opioids, or phencyclidine); personality disorders (such as borderline personality disorder) impulsive disorders, such as maladaptive aggression; bipolar disorders and hyperactive-impulsive attention deficit/hyperactivity (AD/HD) and abuse and addiction (for example alcohol, amphetamine, cocaine or opiate addiction).

### 2. Mood disorders and mood episodes

Major depressive disorder and episodes, manic, mixed and hypomanic mood episodes, depressive episodes with atypical, catatonic or melancholic features, depressive episodes with postpartum onset premenstrual dysphoric disorder, minor depressive disorder, post traumatic, acute stress disorder, obsessive-compulsive disorder and patients with eating disorders.

### 3. Anxiety disorders and episodes of anxiety

Chronic anxiety disorder, panic disorder, agoraphobia, specific phobia, social phobia and generalized anxiety disorder.

### 4. Movement disorders primarily associated to malfunction of basal ganglia:

Different subtypes of dystonia, such as focal dystonias, multiple-focal or segmental dystonias, torsion dystonia, hemispheric, generalised and tardive dystonias (induced by psychopharmacological drugs). Focal dystonias include cervical dystonia (torticolli), blepharospasm (cramp of the eyelid), appendicular dystonia (cramp in the extremities, like the writer's cramp), oromandibular dystonia and spasmodic dysphonia (cramp of the vocai cord) and paroxysmal dystonia.

The compounds of formula (I) have been described for the first time in WO 97/0931, as substances suitable for treating epileptic disorders. Surprisingly, it has now been found that these substances can also be used for an efficient treatment or prevention of central nervous system disorders like the above mentioned disorders but not limited thereto. The compounds can be used for the treatment of mammals and especially for human use.

The number of CH₂ groups of the compounds used according to the invention is either 0 (1-arylimidazolin-2-ones) or 1 (1-aralkylimidazolin-2-ones). Examples of compounds of the formula (I) include :
1-phenyl-4-morpholinoimidazolin-2-one,
1-(4-methoxy)-4-piperidinoimidazolin-2-one,
1-(4-chlorophenyl)-4-morpholinoimidazolin-2-one,
1-(4-chlorophenyl)-4-piperidinoimidazolin-2-one,
1-(4-chlorophenyl)-4-dimethylaminoimidazolin-2-one,
1-(4-bromophenyl)-4-morpholinoimidazolin-2-one,
1-(3-chlorophenyl)-4-morpholinoimidazolin-2-one,
1-(4-chlorophenyl)-4-hexamethyleneimidazolin-2-one,
1-(4-methylphenyl)-4-morpholinoimidazolin-2-one,
1-(4-chlorophenyl)-4-(cyclohexylmethylamino)imidazolin-2-one,
1-(4-fluorophenyl)-4-morpholinoimidazolin-2-one, and
1-benzyl-4-morpholinoimidazolin-2-one.

The substances used in the method of the present invention can be prepared by the process described in US 5,869, 481.

An especially preferred compound to be used as pharmaceutical according to the present invention is 1-(4-chlorophenyl)-4-piperidinoimidazolin-2-one (ELB139; IB-Nomenclature: 1-(4-chlorophenyl)-4-piperidin-1-yl-2,5-dihydro-1H-imidazolin-2-one).

The administration of at least one compound of formula (1) and especially 1-(4-chlorophenyl)-4-piperidinoimidazolin-2-one can be effected in the usual way of administration of psychoactive drugs.

The compounds are preferably administered in form of a pharmacological composition in an amount of 1-100 mg/kg body weight of the patient per day. If inhalative or intranasal administration is chosen, the preferred amount to be administered is 0.05 to 5 mg/kg body weight of the patient. For the use within the framework of the treatment of schizophrenia and other psychotic disorders an administration of 2 to 70 mg/kg body weight is more preferred and an amount of 5-50 mg/kg body weight is especially preferred whereas within the framework of the treatment of dystonia, an amount to be administered of 1-20 mg/kg body weight is more preferred, and administration in an amount of 5 to 15 mg/kg body weight is especially preferred.

In a preferred embodiment, the compounds are administered orally or via an injection in a suitable parenteral formulation, per inhalation, intranasally or as suppositorium.

Further, the compounds are preferably used in connection with common pharmaceutical carriers, excipients or auxiliaries. The application forms are not critical within the framework of the present invention as long as sufficient absorption of the active ingredient is guaranteed.

Further, the compounds are administered as sole treatment for the described diseases and disease stages or in combination with other compounds useful for the treatment of said diseases or disease stages. The combination may be a coadministration using separate administrations for each drug or in form of a fixed combination as mixture with common pharmaceutical excipients or auxiliaries. The application forms of the combinations are not critical within the framework of the present invention as long as sufficient absorption of the active ingredient is guaranteed.

The attached examples of the use according to the invention of 1-ar(alk)ylimidazolin-2-ones clearly prove that the methods according to the invention are extremely efficient when treating psychotic diseases and practically no side effects were observed. The compounds used according to the invention are very well tolerated and can easily be formulated in compositions for the therapeutic or prophylactic application.

A further subject-matter of the present invention is therefore a pharmaceutical composition for the treatment or prophylaxis of central nervous system disorders containing an effective amount of at least one 1-ar(alk)ylimidazolin-2-one of formula (I) 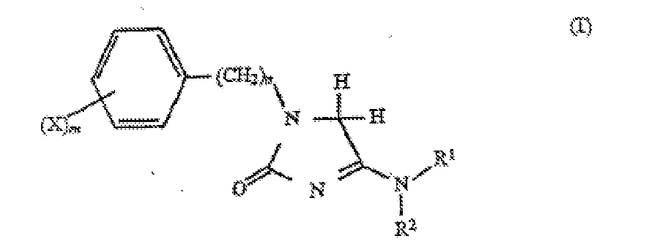 in which X is hydrogen, a C₁₋₄-alkyl, C₁₋₄ alkoxy, trifluoromethyl, or a halogen residue, R¹ and R² are independently of each other a C₁₋₄-alkyl, C₃₋₁₀ cycloalkyl or C₃₋₁₀ heteroalkyl residue, or R¹ and R² are together a C₂₋₆ akylene residue in which a -CH₂-group is optionally replaced by oxygen, nitrogen or sulfur, n is 0 or 1, and m is 0 or a cardinal number from 1 to 5 to a patient in need thereof.

Most preferably, the pharmaceutical composition contains 1-(4-chlorophenyl)-4-piperidinoimidazolin-2-one (ELB 139) as active agent.

The pharmaceutical composition according to the invention furthermore can contain suitable excipients, auxiliaries or filling agents and/or substances, which are necessary or advantageous for the formulation of a suitable form of application. The pharmaceutical composition according to the invention contains the active ingredient (s) preferably in an amount of 1-100 mg/kg body weight of the patient and is intended for the administration per os or parenterally (e.g. intravenously, intramuscularly or subcutaneously).

More preferably, the composition contains the active ingredient in amounts of 25 to 70 mg/kg body weight or 5 to 15 mg/kg body weight respectively, depending of the intended use.

According to a further subject of the present invention, the object was solved by providing a method of treating or preventing central nervous system disorders including psychotic disorders, movement disorders and/or psychotic symptoms associated to other mental disorders and especially for treating anxiety disorders. Said method comprises administering an effective amount of at least one substance which is a subtype selective agonist of benzodiazepine receptors carrying the alpha 3 subunit, however, is not active, that means it does not exert a significant positive GABA increasing effect on receptors carrying the alpha 2 and/or alpha 4 subunit of the GABA receptor, regardless of whether it binds to this receptor or not.

Benzodiazepine receptor ligands which are selective for the alpha 3 subunit of the benzodiazepine receptors can be expected to be effective for treating in the above mentioned CNS diseases.

According to the present invention, selectivity is defined as an at least 20 fold difference in concentration needed to elicit a 50% maximal GABA potentiating response as displayed in the examples and the methods section therein, i.e. an at least 20 fold difference in EC50. Selectivity can also be defined as at least 20 fold difference in binding affinity (an at least 20 fold higher affinity as determined using standard binding experiment procedures) if the binding translates to a functional agonistic effect on the respective receptor subtype. Especially preferred are compounds which are subtype selective for receptors carrying the alpha 3 subunit GABA receptor. Also preferred are alpha 3 subtype selective compounds which are not at all active (which do not exert a significant positive GABA increasing effect) on receptors carrying the alpha 2 and alpha 4 subunit of the GABA receptor regardless of whether they bind to this receptor or not. Especially preferred are also such compounds if they exert the above mentioned features and in addition act as partial agonists for GABA receptors carrying the alpha 3 subunit and in addition act as low affinity agonists or partial agonists (with an at least 20 fold separation in affinity), again with special preference to the partial agonists, with low affinity to the alpha 1 and/or alpha 5 subunit. 1-ar(alk)ylimidazolin-2-one compounds of Formula I as above defined are compounds that show the desired selectivity. An especially preferred example of a compound fulfilling the selectivity criteria as defined above is 1-(4-chlorophenyl)-4-piperidinoimidazolin-2-one (ELB139).

Unexpectedly, it was found that ELB139 and other substances which are comprised by the present invention act as very subtype selective agonists for the benzodiazepine receptor on receptors carrying the alpha 3 subunit. The nature of the effect on these receptors is a partial agonistic one indicating that these compounds and especially ELB139 act as subtype selective partial agonists for receptors carrying the alpha 3 subunit. Also, the compounds and especially ELB139 were active on receptors carrying the alpha 1 or alpha 5 subunit, however at more than 20 times higher concentrations compared to the alpha 3 subunit. Further more, it was found that un-expectedly the compound was not at all active on receptors containing the alpha 2 subunits. Like other benzodiazpines ELB139 was also not active on receptors carrying the alpha 4 subunit. Thus ELB139 acts as a subtype selective compound activating alpha 3 containing receptors and with more than 20 fold less potency alpha 1 and 5 containing receptors and de-selecting alpha 2 and 4 containing receptors. On all receptors showing sensitivity for ELB139 the compound exerted a less strong potentiation of the GABA induced current compared to Diazepam which is indicative of a partial agonistic property. In this respect, partial agonistic activity was concluded if the maximal potentiation of the GABA induced effect was lower at the highest concentration tested as compared to the maximum effect induced by diazepam as positive reference compound at a supramaximal concentration of 1 to 10 µM. The relative partial agonism was in the range of 50 to 70%. Due to the subtype selectivity of the present compounds and especially of ELB139 being selective for the alpha 3 subunit the compounds could be used to evaluate the role of alpha 3 subunits with respect to physiology and pharmacology. ELB139 was found to induce an increase of the serotonine levels in rat brain, however the mechanism of this effect at first remained unclear. To further clarify this effect, it was tested whether this was related to an action of serotonine transporters, i. e. to the mechanism of drugs limiting the function of this transporter and thus resulting in an increase in serotonine (selective serotonine re-uptake inhibitors, SSRIs) or to the now known selective interaction with the alpha 3 benzodiazepine receptor. The compounds of the present invention and especially ELB139 had no effect on the uptake of serotonine in a synaptosomal preparation. On the other hand, the effect of ELB139 on the extracellular level serotonine could be fully blocked and even reversed by administration of the specific benzodiazepine receptor blocker flumazenil which blocks interaction of benzodiazepines with all benzodiazepine sensitive receptors including the one on the alpha 3 subunit. Due to the subtype selectivity these data indicate clearly and unexpectedly that the effect of ELB139 on the serotoninergic system is mediated by the benzodiazepine receptor on atpha 3 subunits. Diazepam is reported to have no effect on serotonine levels if administered in similar experiments indicating that only subtype selective compounds can exert this effect. Thus, based on these data it is now concluded that the alpha 3 subunit is a unique and new target for diseases involving a reduced function of the serotoninergic system or for diseases where an increased function of the serotoninergic system is desired, such as mood disorders including depression and anxiety.

In a second approach, it was tested whether the psychopharmacological behavioural effects of ELB139 which were not typical for benzodiazepines could be reversed by flumazenil administration. The antidepressant effect can be easily linked to the increase in serotonine levels in the brain. All drugs increasing serotonine do exert anti-depressant effects including drugs like fluoxetine, an SSRI. Since the increase in serotonine could be prevented by flumazenil administration, the anti-psychotic effect was selected as most appropriate to evaluate the role of the alpha 3 subunit in the psychopharmacological profile of ELB139. Benzodiazepines without selectivity for the alpha 3 subunit are known to not exert antipsychotic effects. If flumazenil was administered in combination with ELB139, unexpectedly the anti-psychotic effect of ELB139 could be antagonized. These data indicate that not only the alpha 3 subunit is an ideal target for diseases involving the serotoninergic system as described above, but also for other CNS diseases such as psychosis. In addition, these findings indicate that ELB139 and other substances which are selective for alpha 3 subunit containing receptors are potent anxiolytics, anti-dystonics and anticonvulsants. This broad spectrum of activity is in contrast to the subtype selectivity and the discussed linkage of other than the alpha 3 subunit to different diseases. For example, the anticonvulsant activity was so far related mainly to the alpha 1 subunit, but ELB139 acts as a very potent anxiolytic despite the low affinity to the alpha 1 subunit. Similarly, the alpha 2 subunit was seen as pre-dominant for anxiolytic effects with only additive contribution of the alpha 3 subunit for anxiety. However, ELB139 is not at all active on alpha 2 subunits being selective for the alpha 3 subunit. These data further indicate, that the alpha 3 subunit and the benzodiazepine receptor on alpha 3 subunits is an ideal target for the treatment of these diseases including anxiety and epilepsy.

In summary, the body of data, i. e. the effects in vivo in models of psychosis, depression, dystonia, epilepsy and anxiety as well as the effect on serotonine levels indicative for antidepressant activity, in combination with subtype selective activity, open up the potential, that all benzodiazepine receptor ligands which are selective for the alpha 3 subunit of the benzodiazepine receptors can be expected to be active in the above mentioned CNS diseases.

Therefore, another subject-matter of the present invention is a pharmaceutical composition containing a benzodiazepine receptor ligand which is selective for the alpha 3 subunit of the benzodiazepine receptors. Such substances with high selectivity for the alpha 3 subunit can easily be detected using well established and described screening systems. Such systems can comprise receptor binding assays as a first step, but using binding assays which however have to be based on membrane fractions containing the respective GABA receptor subunits. Such preparations can be obtained from cell lines expressing and assembling after stable of transient transfection the functional GABA receptor complex consisting of the alpha subunit under investigation, i. e. alpha 1, 2, 3, 4, or 5, in combination with one beta subunit (perferably the beta 2 subunit and one gamma subunit, preferably the gamma 2 subunit. A different source for the GABA receptors sybtypes can be obtained from expression systems expressing the recombinant proteins of the different subunits. Such expression systems may be bacteria, yeasts or eukaryotic cells. Using such binding assays, compounds with high affinity for the alpha 3 subunit and high selectivity over the other GABA receptors containing the alpha subuntis 1, 2, 4 or 5, can be easily identified. A radioligand may be 3(H)-Flunitrazepam or other well described radioligands without selectivity for individual GABA subunits.

Since benzodiazepine receptor ligands can act as agonists, neutral ligands (antagonists) and inverse agonists, a functional assay is needed to identify agonists as well as partial agonists. Such assays may be a modified binding assays, using the binding of 3(H)-Muscimol as read out, since agonists as well as antagonists and inverse agonists affect differentially the binding characteristics of Muscimol.

A different functional assays which can be used to identify the intrinsic activity of benzodiazepine ligands is based on electrophysiological techniques using either Xenopus oocytes as expression systems or transfected cell lines such as CHO cells transfected with the respective alpha, beta and gamma subunits as described elesewhere. Again another functional assay may be based on transfected cell lines exposed to GABA and the compound to be tested, but using the membrane potential as read out for the receptor interaction. The result of such a stepwise screening approach is a compound which has high selectivity for the alpha 3 subunit and which may act as a full or partial agonist at the alpha 3 subunit.

Such a receptor ligand compound may also act as a partial agonist with at least 20 fold lower affinity to the alpha 1 and 5 containing GABA receptor compared to the alpha 3 containing GABA receptor. Preferably such pharmaceutical composition contains an 1-ar(alk)ylimidazoline-2-one of Formula I as defined above which shows the desired selectivity. Preferably, such pharmaceutical composition contains the compound 1-(4-chlorphenyl)-4-piperidinoimidazolin-2-ones (ELB139). Further, it is possible and sometimes advisable that the pharmaceutical composition contains further excipients or auxiliaries and the composition can be either prepared for parenteral or for oral administration. Like described above, a dose of 1-100mg/kg body weight of the patient has been found to be an effective dose of the active compound. Further preferred dosages contained in the pharmaceutical composition are 2 to 70 mg/kg body weight or 5 to 50 mg/kg body weight for treatment of schizophrenia and other psychotic diseases, and 1 to 20 mg/kg or 5 to 15 mg/kg body weight for treatment of dystonia.

Such pharmaceutical composition is useful for treatment of central nervous system disorders and preferably for treating psychosis, depression, dystonia, epilepsy and anxiety as described above in more detail.

Such compounds or compositions including ELB139 are also claimed to exert selective positive effects on CNS disorders which can currently not be treated with the available benzodiazepine receptor ligands, i.e. depression, psychosis, dystonia and related CNS diseases with a special focus on diseases which can be treated with compounds exerting an effect on serotonine tevets in the brain including depression. Such compounds are also claimed to exert effects in diseases which can be currently treated with benzodiazepine receptor ligands but at a better side effect profile reducing the amnestic, sedative, hypnotic, addiction inducing, muscle relaxant, and CNS-depressant effect of benzodiazepine recpetor ligands and the development of tolerance. Such diseases include anxiety disorders, epilepsy, sleep disorders, and other diseases responsive to benzodiazepine treatment.

The invention is further explained by the following Examples and Figures:

The Figures show:
**Figure 1****:** Activity, total distance travelled, stereotyped sniffing, other stereotypies and ataxia stimulated by 0.2 mg/kg MK-801 administered i.p. 10 minutes prior to test in female rats.
   Haloperidol was administered i.p. and E131-00139 was administered p.o. 1 hour prior to test. * significant to control p<0.05, ** significant to control p<0.01, *** significant to control p<0.001, # significant different to each other p<0.05.
**Figure 2****:** antidystonic activity of E131-00139
   At a lower dose, i.e. at 5 mg/kg i.p., still a significant antidystonic activity was present.
**Figure 3****:** Effect of E131-00139 on 5-HT release in the striatum of rats
   30 mg/kg E131-00139 was administered i.p.. Data are shown as percent of mean basal level of 5-HT (mean SEM); * = significant to tylose, p<0.05.
**Figure 4****:** Effect of E131-00139 on dopamine release in the striatum of rats
   30 mg/kg E131-00139 was administered i.p.. Data are shown as percent of mean basal level of dopamine (mean SEM).
**Figure 5****:** Whole cell recordings of HEK 293 cells expressing recombinant rat aiβ2γ2 (i=1-5) GABA_{A} receptors. Currents were normalized to the GABA concentrations used in the absence of all different drugs. Increasing concentrations of diazepam (▲), diazepam + 10 µM Ro15-1788 (Δ), E131-139 (□), or E131-139 + 10 µM Ro15-1788 (□) were co-applied with GABA concentrations about EC₂₀. Error bars indicate the standard error of mean (±SEM) for 4 cells each.
**Figure 6****:** Effects of ELB139 (= E131-00139, 10+30 mg/kg) on the time spent immobile, the duration of swimming and the time span the animals tried to climb out in the rat forced swim test compared to vehicle treated controls. (* p<0.05, One Way ANOVA followed by the Holm-Sidak method). Data are presented as mean ±SEM (n=10).
**Figure 7****:** Effects of fluoxetine (10+30 mg/kg) on the time spent immobile, the duration of swimming and the time-span the animals tried to climb out in the rat forced swim test compared to vehicle treated controls. (* p<0.05, One Way ANOVA followed by the Holm-Sidak method) Data are presented as mean ±SEM (n=10).
**Figure 8****:** Effect of haloperidol on MK-801-induced psychosis-related behaviour in female rats
   Two separate trials are shown. Haloperidol (H) at 0.5 mg/kg i. p. was administered 60 min prior to test when given alone and 30 min prior to test when given in addition to flumazenil (F). Flumazenil at 5 mg/kg i. p. was administered 20 min prior to test. MK-801 at 0.1 mg/kg i. p. was given 10 min prior to test. Data are shown as mean ±SEM. Significant to control (C): * p<0.05, ** p<0.01, ***p<0.001.

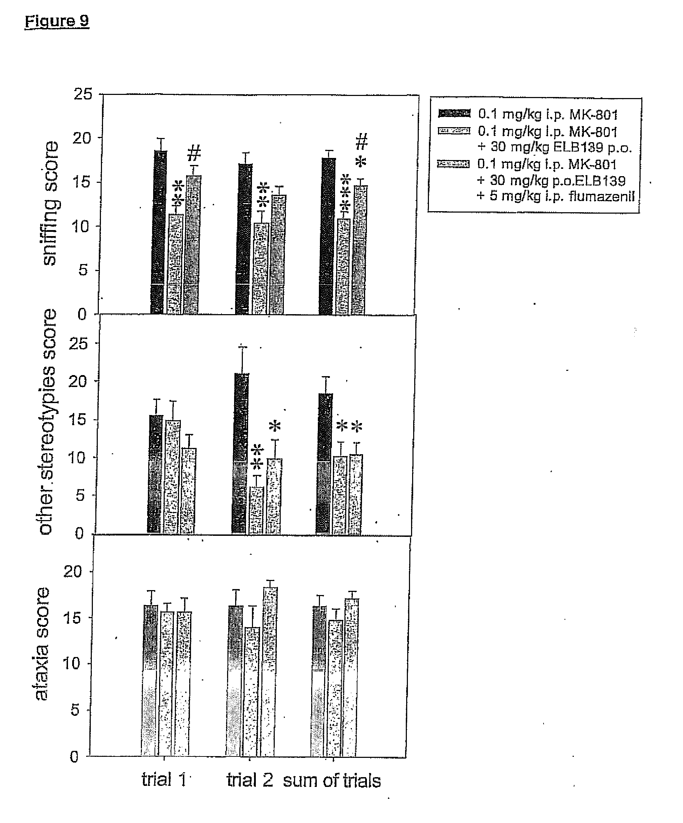

**Figure 9****:** Effect of ELB139 on MK-801-induced psychosis-related behaviour and its reversibility by flumazenil in female rats
   ELB139 at 30 mg/kg p.o. was administered 1 h prior to test. Flumazenil at 5 mg/kg i.p. was administered 20 min prior to test. MK-801 at 0.1 mg/kg i.p. was given 10 min prior to test. Effect on MK-801-induced stereotyped sniffing, other stereotypies and ataxia are shown as mean ±SEM. Significant to control: * p<0.05, ** p<0.01, ***p<0. 001; significant to ELB139 group: # p<0.05.

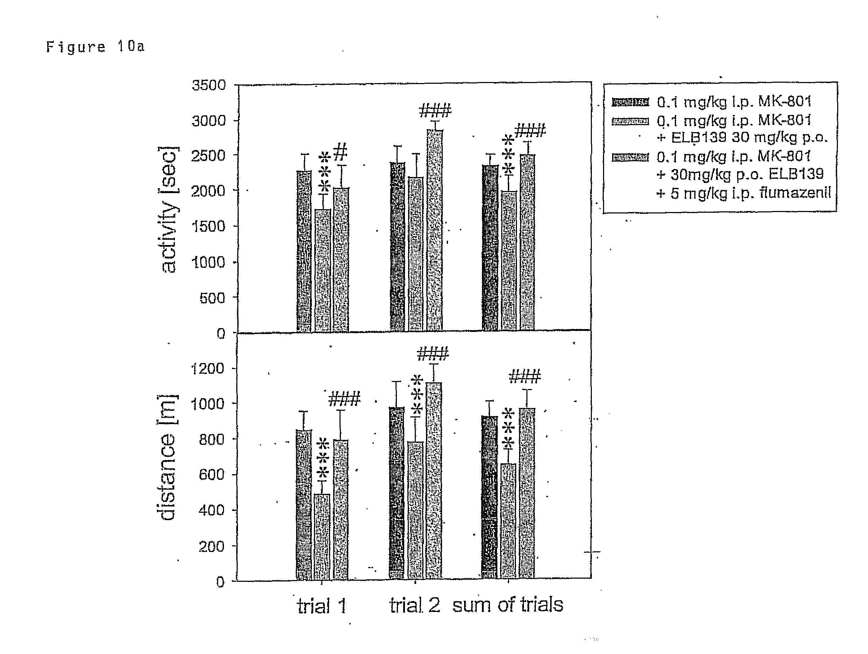

**Figure 10a****:** Effect of ELB139 on MK-801-induced psychosis-related behaviour and its reversibility by flumazenil in female rats
   ELB139 at 30 mg/kg p.o. was administered 1 h prior to test. Flumazenil at 5 mg/kg i.p. was administered 20 min prior to test. MK-801 at 0.1 mg/kg i.p. was given 10 min prior to test. Effect on MK-801-increased activity and distance travelled are shown as mean ±SEM. Significant to control:* p<0.05; significant to ELB139 group: # p<0.05.

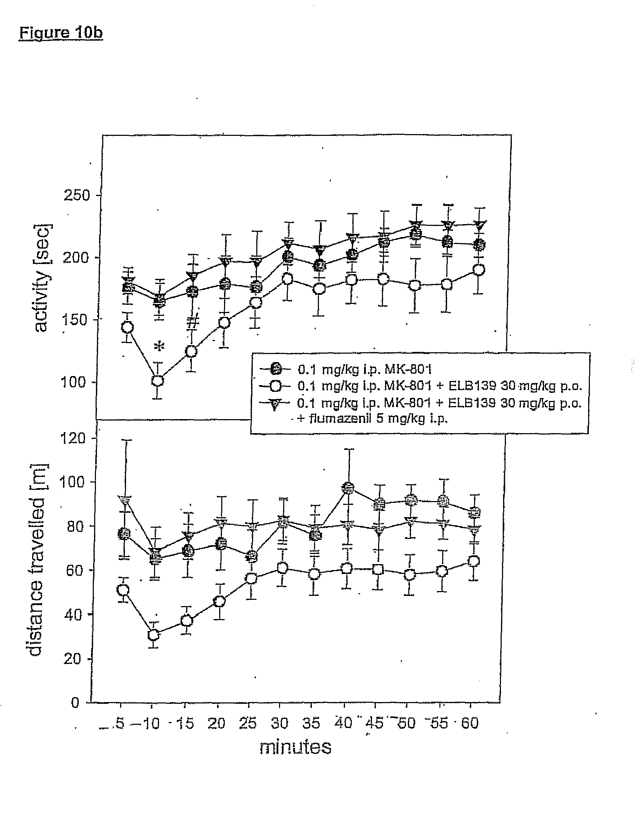

**Figure 10b****:** Effect of ELB139 on MK-801-induced psychosis-related behaviour and its reversibility by flumazenil in female rats
   ELB139 at 30 mg/kg p.o. was administered 1 h prior to test. Flumazenil at 5 mg/kg i.p. was administered 20 min prior to test. MK-801 at 0.1 mg/kg i.p. was given 10 min prior to test. Effect on MK-801-increased activity and distance travelled are shown in 5-minute-intervalls as mean ± SEM. Significant to control:* p<0.05; significant to ELB139 group: # p<0.05.

### Examples

### 1. Treatment of psychotic disorders

### 1.1. Animals

Female Wistar rats (Crl: (WI) BR, Charles River, Sulzfeld, Germany) weighing 150 to 180 g were used for the experiment. They were housed under standard conditions in groups of five on a 12 h light/dark cycle (light on at 0600 h) with ad libitum access to food (Pellets, ssniff M/R 15, Spezialdiat GmbH, Soest/Westfalen) and water.

### 1.2. Chemicals

E131-00139 1-(4-chlorphenyl)-4-piperidinoimidazolin-2-one, MW 277.75) was manufactured by elbion AG. Haloperidol (4-(4-[4-chlorophenyl]-4-hydroxy-1-piperidinyl)-1-(4-fluorophenyl)-1-nutanone, MW 375,9) was obtained by ratiopharm GmbH, Ulm, Germany, MK-801 (dizocilpine, MW 337.37) was obtained by Tocris, distributed by Biotrend Chemikalien GmbH, Köln, Germany. All other chemicals used were obtained from Sigma-Aldrich Chemie GmbH, Germany or from Merck, Germany.

### 1. 3. Drug administration schedule/dosage

Applied volume: 0.5 ml/100g

| **substance** | **Number of animals** [n] | **Dosage** [mg/kg] | **pre-treatment** [min] | **number of application** [n] | **Route of administration** |
|---|---|---|---|---|---|
| MK-801 | 17 | 0.2 | 10 | 1 | i.p. |
| E131-00139 | 6 | 30,60 | 60 | 1 | p.o. |
| haloperidol | 10 | 0.5 | 60 | 1 | i.p. |

### 1.4. Preparation of compounds:

E131-00139 was freshly suspended in 0.5% hydroxyethylcellulose so that an administration volume of 0.5 ml/100 g was reached for each substance and dose. Haloperidol injection solution was diluted with saline so that an administration volume of 0.5 ml/100 g was reached. The suspensions were placed on a magnetic stirrer before and during dosing procedures. Hydroxyethylcellulose was solved in distilled water.

### 1.5. Experimental procedure

The behaviour induced by the NMDA antagonist MK-801 is generally accepted as a rat model of psychosis. MK-801 induces stereotypies, hyperactivity and ataxia in rats after intraperitoneal administration.

Locomotor activity of the rats is recorded by the MotiTest Apparatus (TSE, Bad Homburg, Germany). The test area consisted of a squared arena (45 (45 cm) with protective plexiglass walls (20 cm of height) where rats could freely move. Horizontal movements were recorded by 32 infrared photocells arranged along the bottom of each wall of the arena. Vertical movements (rearing) were recorded by a horizontal row of 32 infrared photocells 12 cm above the floor. The following parameters are measured by the computer program "ActiMot" (TSE, Bad Homburg, Germany):
active time [s] and total distance travelled [m]

Stereotypies, divided into stereotyped sniffing and other stereotypies, and ataxia are scored by the experimenter every five minutes for one hour (12 intervals) according to the method described by Andiné et al. (1999). The scores of the 12 intervals are added for each parameter.

| score | stereotyped sniffing | other stereotypies | ataxia |
|---|---|---|---|
| 0 | no stereotyped sniffing | no other stereotypies | normal body control |
| 1 | discontinuous sniffing (free interval > 5 s) | discontinuous stereotypies (free interval > 5 s) | falling tendency upon movement |
| 2 | continuous sniffing | continuous stereotypies | falling upon movement |
| 3 | - | - | Almost unable to move |

The day of experiment the female rats are placed in the laboratory and receive the test compound, the reference substance or vehicle at the appropriate time prior to test. MK-801 0.2 mg/kg is intraperitoneally administred 10 minutes prior to test.

At the beginning of the test the rats are placed in the centre of the squared arena of the MotiTest apparatus. Behaviour of the rats is recorded for one hour. After each run animals are removed and the boxes thoroughly cleaned and dried.

### 1.6. Statistics

Results were analysed by one way analysis of variance (ANOVA). Tukey test was used for individual comparison. P (0.05 was regarded as significant.

### 1.7. Results

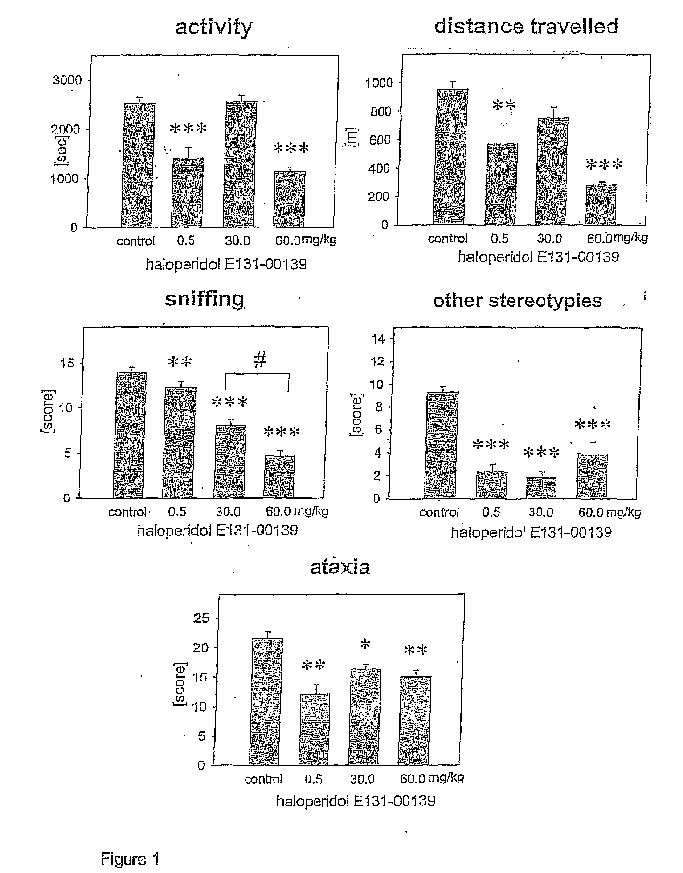

The results of the test are shown in figure 1. Haloperidol significantly reduced all symptoms induced by MK-801 [p<0.001] as described by Andiné et al. (1999).

At 30 mg/kg p. o. E131-00139 significantly reversed the stereotyped sniffing, the other stereotypies and the ataxia induced by MK-801 and distinctly reduced the total distance travelled increased by MK-801. At this dose it did not reduce MK-801-stimulated activity.

At 60 mg/kg p. o. E0131-00139 significantly reduced all parameters induced by MK-801.

The stereotyped sniffing was reduced dose dependently [p<0.05]

### 2. Treatment of dystonia

### 2. 1. Animals

Experiments were carried out in male and female dt^{sz} mutant Syrian golden hamsters which were obtained by selective breeding as previously described in detail (Fredow and Loscher, 1991). They were housed under standard conditions in groups of three to five on a 12 h light/dark cycle (light on at 0600 h) with ad libitum access to food (Altromin 1320 standard diet, Altromin, Lage, Germany) and water.

### 2. 2 Chemicals

E131-00139 1-(4-chlorphenyl)-4-piperidinoimidazolin-2-one, MW 277.75) was manufactured by elbion AG. All other chemicals used were obtained from Sigma-Aldrich Chemie GmbH, Germany or from Merck, Germany.

### 2.3. Drug administration schedule/dosage

Applied volume. 0.2 ml/20g

| **substance** | **Number of animals** | **Dosage** | **observation period after administration** | **number of application** | **Route of administration** |
|---|---|---|---|---|---|
| | [n] | [mg/kg] | [min] | [n] | |
| E131-00139 | 8 | 5 | 180 | 1 | i.p. |
| E131-00139 | 8 | 10 | 180 | 1 | i.p. |

### 2. 4. Preparation of compounds

E131-00139 was freshly suspended in 0.5% hydroxyethylcellulose so that an administration volume of 0.2 mol/20 g was reached for each substance and dose. The suspensions were placed on a magnetic stirrer before and during dosing procedures. Hydroxyethylcellulose was solved in distilled water.

### 2. 5. Experimental procedure

Drug testing was performed at the maximal sensitivity of the hamsters for induction of dystonic attacks, i. e. between 30 and 40 days of age. Dystonic attacks were induced by a triple stimulation technique: The hamsters were taken from their home cage and placed on a balance (to determine body weight), were then injected i.p. with saline (control) or drug, and immediately placed individually in a new and empty plastic cage. Dystonic attacks started within a few minutes after placing the hamsters in the new plastic cage. The animals were observed in this cage for 3 h, and the severity of the dystonic movements was rated for the time period 0-1 hour, 1-2 hours and 2-3 hours as follows, always rating the maximum stage reached within the observation period:
- Stage 1: flattened ears and flattened posture while walking
- Stage 2: facial contortions, rearings with forelimb crossing, disturbed gait with retarded setting of forepaws
- Stage 3: stiffened hindlimbs, so that the animals appear towalk on tiptoes in a dysmetric hypergait
- Stage 4: loss of balance
- Stage 5: hindlimbs hyperextended caudally, animals continue to pull itself with the functional forelimbs
- Stage 6: animals immobilised in a twisted, hunched posture with both hindlimbs and forelimbs tonically extended forward, erected (Starub- like) tail, alternating unilateral forelimb elevation, head weaving, and opisthotonus

The final stage persisted for two to five hours, but rapid recovery occurred thereafter. All mutant hamsters did not progress through the entire sequence described; the individual maximum stage was usually reached after 45-170 minutes.

### 2.6. Statistics

Results were analysed by Friedman-test followed by Wilcoxon-test. P < 0.05 was regarded as significant.

### 2. 7. Results

A first group of animals (n=8) was tested for ocurrence and severity of dystonic attacks at the age of 32-33 days after administration of vehicle (i.p.) as well as after performing the triple stimulation procedure as described above. Two or three days later, the same animals received E131-00139 i.p. and were again observed for 3 hours and again 2-3 days later the animals were re-tested after vehicle administration to serve as post dose control. The results of this test are displayed in figure 2. In this graph, three stacks of three bars each are displayed. The first stack represents the first hour of observation staring with drug administration, the second stack of bars represents the second hour of observation and the third stack the third hour.

In each stack 3 bars are displayed. The first (open) bar represents the control response of the animals recorded 2-3 days prior to the drug test, the black bar represents the results obtained after drug administration and the third (gray) bar represents the post drug control tested 2-3 days after the drug administration. The pre-and post test was used to exclude that a reduction of severity of stages was only due to a reduction in sensitivity for the induction of dystonic attacks is age dependent.

In the current experiment E131-00139 was shown to exert a potent antidystonic effect. At the dose tested, i. e. 10 mg/kg i.p. , the compound was well tolerated and no sedation was observed. During the total observation period the severity of the dystonic attack was significantly reduced inidcating a long duration of action. The remaining symptoms, i. e. on average stage 2.3 to 2.4, are the stages which are reached within minutes after start of the experiment, i. e. at a time where no or only minimal plasma levels of E131- 00139 are present.

### 3. Anxiolytic and antidepressive activity

To further characterize E131-00139 the compound was investigated by microdialysis. The extracellular concentration of two neurotransmitters, serotonin (5HT) and dopamin, and their metabolites were determined in the striatum.

### 3.1. Animals

Male Wistar rats (Cri: (WI) BR, Charles River, Sulzfeld, Germany) weighing 200 to 260 g were used for the experiment. They were housed under standard conditions in groups of five on a 12 h light/dark cycle (light on at 0600 h) with ad libitum access to food (Pellets, ssniff M/R 15, Spezialdiat GmbH, Soest/Westfalen) and water.

### 3.2. Chemicals

E131-00139 1-(4-chlorphenyl)-4-piperidinoimidazolin-2-one, MW 277.75 was manufactured by elbion AG. All other chemicals used were obtained from Sigma-Aldrich Chemie GmbH, Germany or from Merck, Germany.

### 3.3. Drug administration schedule/dosage

Route of application: i.p.
Applied volume: 0.5 ml/100g

| substance | Dosage [mg/kg] | pre-treatment time [min] | number of application [n] |
|---|---|---|---|
| E131-00139 | 30 | 0 | 1 |

### 3.4. Preparation of compounds suspended in 0.5% hydroxyethylcellulose containing 10% polyethyleneglycol 300 (PEG300)

E131-00139 was freshly suspended in 0.5% hydroxyethylcellulose so that an administration volume of 0.5 ml/100 g was reached for each dose. The suspensions were placed on a magnetic stirrer before and during dosing procedures. Hydroxyethylcellulose was dissolved in distilled water.

### 3.5. Experimental procedure

### Surgery

The day before the experiment, male rats were anaesthetised with chloral hydrate (3.6%, Imt/100 g i.p.) and placed in a stereotaxic frame to implant a microdialysis guide cannula (CMA/12, Carnegie Medicine, Sweden). Scalp was incised in the median anterior-posterior direction between lambda and bregma and a small hole was drilled into the skull. The stereotaxic coordinates were AP = +1.0 mm, L = -3.0 mm from bregma and 1.5 mm from the skull surface for the striatum according to the atlas of Paxions and Watson, (1986) (1). Dental cement (Sinfony (2)) was used to fix the guide cannula and anchor screws to the cranium. During the surgery rectal temperature was maintained at 37°C using a heating blanket.

### Microdialysis

The day before the experiment the microdialysis probe (CMA/12, membrane length 4mm, Carnegie Medicin, Sweden) was inserted into the striatum through the guide cannula. On the day of experiment the probe was perfused with Ringer solution (148 mM NaCl, 4 mM KCI, 2,4 mM CaCl₂, pH = 6,0). The flow rate (1 µl/min) allowed to collect 20 µl samples every 20 minutes into microvials. Microvials were stored in a fraction collector (CMA/170) at a temperature of 8°C until analysed. Using a swivel joint the perfusion arrangement allowed the animal to move freely within a hemispherical bowl. After an adjusting time of one hour 3 consecutive 20-min-fractions were collected to establish a stable basal level of neurotransmitter release.

Thereafter 30 mg/kg E131-00139 or an equivalent volume of 0.5 % tylose/PEG300 9: 1, respectively, was administered. Then collecting samples was continued for at least 220 minutes.

### Analysis of dialysates

Dialysates were directly analysed by reverse-phase high-performance liquid chromatography with electrochemical detection (HPLC-EC). The samples were separated by a ZORBAX SB-Aq 2.1 mm ID x 100 mm column (Agilent Technologies). 1 µl 1% perchloric acid were added to the 20-minutes-fraction and 10 µl of this mixture were injected into the HPLC-system.
The mobile phase contained:

| | |
|---|---|
| KH2P04 | 50mM |
| Octan-1-sulfonsäure Natriumsalz (NOS) | 2,2mM |
| EDTA | 0. 086mM |
| 2MH₃PO₄ | 5ml |
| Methanol (MeOH) | 83ml |
| Acetonitril | 10ml |
| | at pH 3.5 |

The method (serotonin) runs at a flow of 0.23 ml/min and a column temperature of 38°C, sample thermostat 8°C.

At night flow was reduced to a flow of 0.1 ml/min. Catecholamines were oxidized at 500 mV (Model 5014 B microdialysis cell, esa). Dopac was measured at 200 nA, dopamine at 2 nA, HIAA at 100 nA, HVA at 2 nA, and serotonin at 500 pA.

In order to calibrate the system external standards with 5 concentrations each were run regularly:

| | |
|---|---|
| DOPAC (3, 4-dihydroxyphenylacetic acid) | 1000, 500, 250, 125, 62.5 nM |
| DOPAMINE (3-hydroxytyramine hydrochloride) | 8, 4, 2, 1, 0.5 nM |
| HIAA (5-hydroxy-3-indoleacetic acid) | 200, 100, 50, 25, 12.5 nM |
| HVA (homovanillic acid) | 800, 400, 200, 100, 50 nM |
| SEROTONIN (5-hydroxytryptamine-kreatininsulfat) | 0.15, 0.075, 0.0375, 0.01875, |
| | 0.009375 nM |

Before implanting and after removing the microdialysis probe a recovery was carried out with a solution containing 2000 nM DOPAC, 40 nM dopamine, 1000 nM HIAA, 2000 nM HVA and 0.8 nM serotonin. The recovery rate of the probes lay between 5 and 20 %.

### Histology

After completion of the experiment the brain of the rats was removed and postfixed in formalin (10%) for approximately 10 days. The brains were cut using vibroslice (TSE) and stained with toloidin blue to prove probes right position.

### Statistics

Basal level of 5-HT release showed interindividual differences. Therefore data of each animal were expressed as percentages. Data from dialysates before administering the substances were averaged, and the mean set as 100 %; all individual values were calculated accordingly.

Results were analysed by two way analysis of variance (ANOVA) with time and drug as the two factors. Tuckey test was used for individual comparison. P (0.05 was regarded as significant.

### 3.6. Results

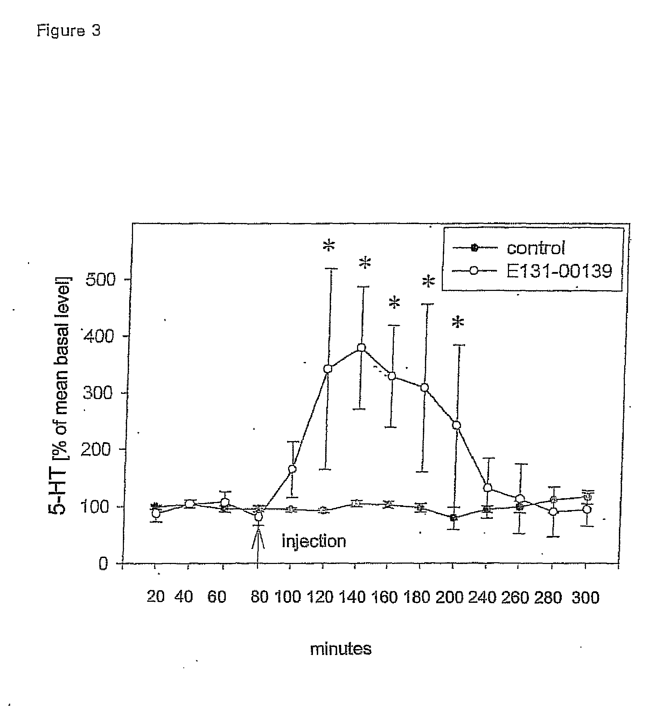

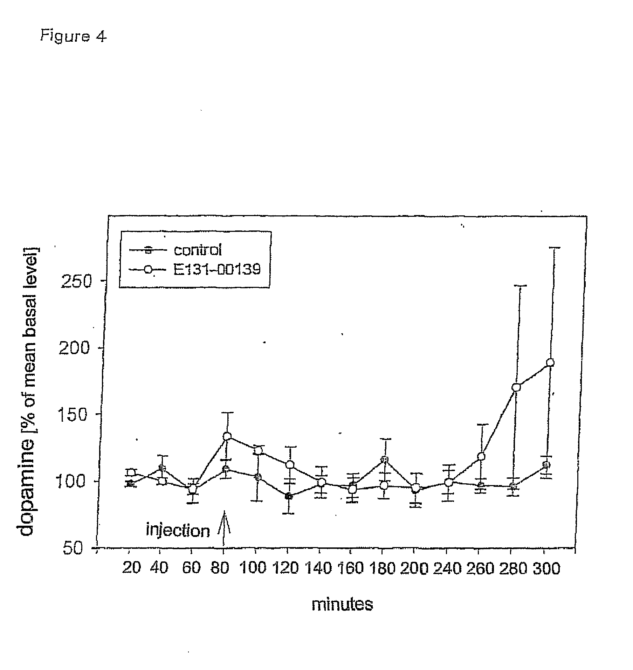

E131-00139 at 30 mg/kg i.p. significantly and unexpectedly increased 5-HT concentration in the striatum of rats for 1 hour and 40 minutes (figure 3) without influencing HIAA concentration, a metabolite of 5-HAT (data not shown). It did also not influence the simultaneously measured dopamine concentration in the striatum of rats (figure 4). Changes of 5-HT levels are indicative of an anxiolytic and antidepressant effect.

The mechanism of this increase is not clear. It cannot be explained by the benzodiazepine-like activity of the compound as benzodiazepines such as diazepam do not increase but rather decrease 5-HT release in the brain (Pei et al., 1989) and by that counteracting to the effect of SSRIs. Additionally, the increase of striatal 5-HT is not an essential and typical mechanism of antiepileptic drugs as the effect of antiepileptics on 5-HT release in the brain is varying depending on the anticonvulsant. Carbamazepine also produce an increase of basal 5-HT release probably by influencing N-type Ca2+ channels (Kawata et al., 2001). Valproate as well enhances extracellular 5-HT (Murakami et al., 2001) whereas phenytoin and gabapentin decrease it (Okada et al., 1997; Taylor et al., 1998).

The effect of E131-00139 on extracellular 5-HT concentration may be compared to that of fluoxetine and other selective serotonin reuptake inhibitors (SSRI) (Li et al., 1996). Thus, E131-00139 opens up new vistas for the treatment of central nervous system disorders as the compound class may combine the long-lasting but late onset activity of drugs increasing extracellular 5-HT concentration in the brain and the direct and rapid onset of the anxiolytic activity of the benzodiazepines which is a well known quality of the compound class 1-Ar(alk)yl-imidazolin-2-ones. By bringing together the advantageous profile of a benzodiazepine agonist and the profile of a SSRI without any risk to alleviate the effect on the serotonin level by the benzodiazepine mediated effect E131-00139 exhibits an exceptional and improved potential for the treatment of chronic anxiety syndromes, panic disorder, agoraphobia, specific phobia, social phobia and generalised anxiety disorder, and depressive disorders, such as major depressive disorder and episodes, manic, mixed and hypomanic mood episodes, depressive episodes with atypical, catatonic or melancholic features, depressive episodes with postpartum onset premenstrual dysphoric disorder, minor depressive disorder, post traumatic and acute stress disorder.

### Example 4

Subtype selective and partial agonistic effect of ELB139 as an example of a subtype selective partial agonistic alpha 3 preferring compound.

The ligand gated ion channels opened by γ-amino butyric acid (GABA_{A} receptors) are pentamers assembling from two to three different subunits out of an array of six α, three β, three γ, a δ, an ε, a π and a θ subunit (see Hevers et al., 1998). Most likely it is the structural heterogeneity of GABA_{A} receptors that forms the basis for their functional diversity. Most benzodiazepines (BZ) recognising the GABA_{A} receptor modulate Cl⁻flux through these receptor channels, thereby affecting synaptic transmission in the CNS. For example, the sedative-hypnotic BZ diazepam, used in the present study as a reference compound, imposes a number of effects on the function of the CNS, resulting in a spectrum of clinical actions ranging from sedation at low doses to induction of anaesthesia at significantly higher doses. Thus, large efforts are made to improve the GABAergic subtype specificity of drugs like the BZs in order to reduce their actions on those neuronal systems not involved in the therapeutic effects.

The GABA_{A} receptor subunit composition determines both affinity and efficacy of the endogenous ligand as well as for drugs like BZs. Variations among the α subunits have profound impact on the affinity and efficacy of distinct classes of BZ ligands, whereas other classes are indiscriminate (Pritchett et al., 1989, Wisden et al., 1991). E.g., diazepam, like most but not all other 1,4-BZs, was previously shown not to differentially bind to receptor subtypes of the general form αiβjγ2 (i = 1 - 3, 5 ; j = 1-3) (Lüddens, 1995, Benavides, 1992, Pritchett, 1990).

The present experiments were conducted to examine in detail the potency and efficacy of the putative BZ receptor ligand ELB139 in the presence and absence of the BZ antagonist flumazenil (Ro15-1788) to a number of GABA_{A} receptor subtypes expressed in the hetero system of human embryonic kidney (HEK 293) cells. The chosen receptor subtypes αiβjγ2 (i = 1-5) are likely to constitute the majority of native GABA_{A}/BZ-receptors. The efficacy, potency, and α subunit specificity of the novel compound was compared to those of diazepam.

### 4. 1. Experimental Section

### Materials

With the exception of ELB139 (E131-00139) all compounds were from commercial sources and in analytical grade.

### 4.2. Cell culturing and cell transfection

For electrophysiological recording HEK-293 cells were passaged and replated on 12-mm glass coverslips located in 9.6-cm plastic dishes filled with 10 ml of Minimum Essential Medium (MEM, Gibco) supplemented with 158 mg/l sodium bicarbonate, 2 mM glutamin (Gibco), 100 U/ml penicillin-streptomycin (Gibco), and 10% foetal calf serum (Gibco). Cultures were maintained at 37°C in a humidified 95% O₂/5% CO₂ atmosphere for 2-3 days.

Transfection with recombinant rat GABA_{A} receptors was carried out as described in detail (Korpi and Luddens, 1993, Luddens and Korpi, 1995b). Briefly, HEK 293 cells were transfected in triple combinations using the phosphate precipitation method with rat GABAA receptor cDNAs in eukaryotic expression vectors (Pritchett, 1990) for the α, β and γ subunits. For optimal receptor expression, final concentrations (µg vector DNA per 9.6 cm tissue culture plate) were: α1, 2; α2 4.8; α3, 1.2; α4, 10; α5, 0.8; β2, 0.4; and γ2S, 0.3. The γ2S variant is abbreviated γ2 in the remainder of the text. To identify transfected cells all subunit combinations were co-transfected with 1 µg per plate of pNI-EGFP.

### 4.3. Electrophysiology

Two days after transfection single coverslips containing HEK 293 cells were placed in a recording chamber mounted on the movable stage of a fluorescence microscope (Olympus IX70) and perfused with a defined saline solution containing (in mM): 130 NaCl, 5.4 KCl, 2 CaCl₂, 2 MgSO₄, 10 glucose, 5 sucrose, and 10 HEPES (free acid), pH adjusted to 7.35 with about 35 mM NaOH. Transfected cells were identified by their green fluorescence due to the expression of the pNI-EGFP vector and ligand-mediated membrane currents of these cells were studied in the whole-cell configuration of the patch-clamp technique (Hamil et al., 1981).

Patch-clamp pipettes were pulled from hard borosilicate capillary glass (0.5 mm ID, 1.5 mm OD, Vitrex, Science Products GmbH, Hofheim, Germany) using a horizontal puller (Sutter Instruments, CA, Model P-97) in a multi-stage process. The pipettes had an initial resistance of 2-4 MΩ when filled with a solution containing (in mM): 90 KCI, 50 KOH, 2 CaCl₂, 2 MgCl₂, 10 EGTA, 3.1 ATP (dipotassium salt), 0.4 GTP (tri-sodium salt), and 10 HEPES (free acid), pH 7.35.

The junction potential between the pipette and the external solution was less than 2.3 mV and therefore neglected. Seal resistances > 1 GΩ were routinely obtained by applying gentle suction to the pipettes. Membrane rupture was monitored electrically as an increase in capacity. Pipette capacitance, membrane capacitance, and series resistance were electronically compensated to achieve minimal capacitative transients. A series resistance compensation of >60% was regularly used.

Using a fast perfusion stepper system (SF-77B, Perfusion Fast Step, Warner Instruments, Inc., Midwest, USA) test solutions containing the approximate receptor subtype specific GABA EC₂₀, GABA EC₂₀ plus increasing concentrations of diazepam (in µM): 0.01, 0.1, 1, 10, and GABA EC₂₀ plus increasing concentrations of ELB139 (in µM): 0.01, 0.03, 0.1, 0.3, 1, 3, 10, 30. In the case of diazepam and ELB139 an experimental set of all concentrations with additionally 10 µM Ro15-1788 was tested. In all cells recorded the presence of the γ2 subunit in the heteropentamere was monitored by the application of 1 µM zolpidem plus the EC₂₀ of GABA before testing the experimental drugs.

Responses of the cells were recorded by a patch-clamp amplifier (EPC-8, HEKA-Electronic, Lambrecht, Germany) in conjunction with a standard personal computer and the pClamp 8.1 software package (Axon Instruments, Foster City, CA). The standard holding-potential for the cells was -40 mV. Whole cell currents were tow-pass tittered by an eight-pole Bessel filter at 5 or 3 kHz before being digitised by a Digidata 1322A interface (Axon Instruments, Foster City, CA) and recorded by the computer at a sampling rate of at least 1 kHz.

### 4.4. Results and Discussion

Efficacy, potency, and GABA_{A} receptor α subunit specificity of the new compound ELB139 was tested with the whole cell configuration of the patch-clamp technique by applying increasing drug concentration together with GABA of the approximate EC₂₀ on green fluorescing cells. BZ binding site specificity of the new compound was tested on the same cells in an identical experimental setting with additionally applying 10 µM Ro15-1788.

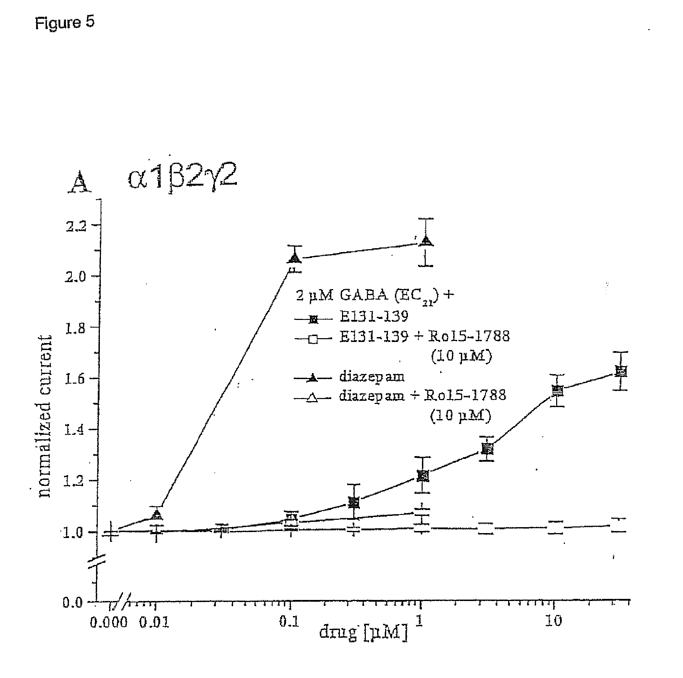

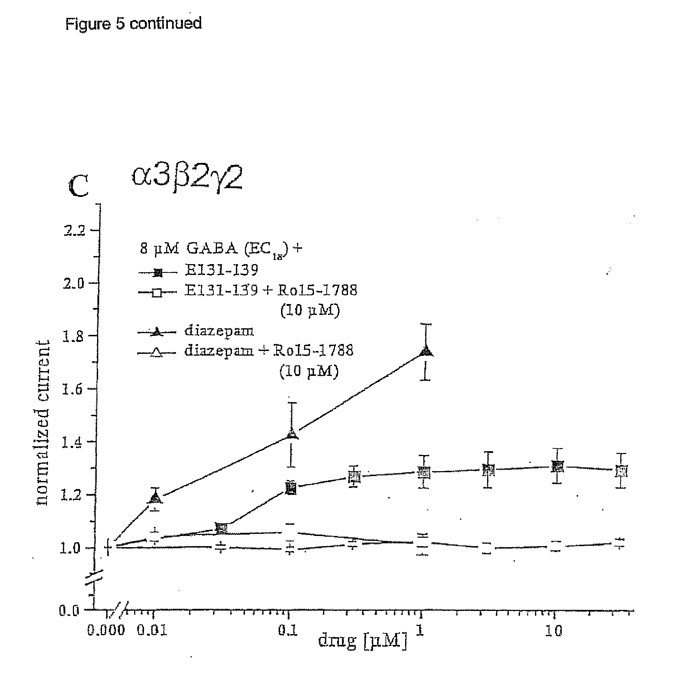

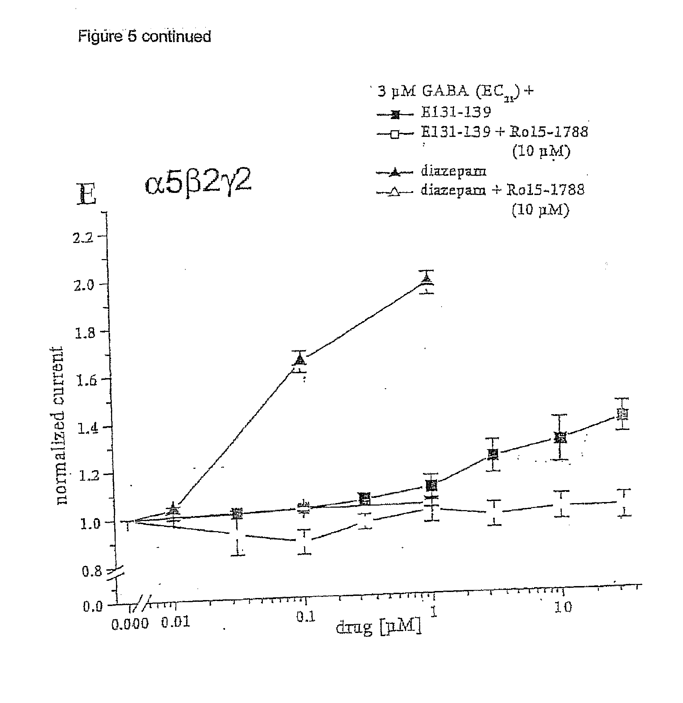

In α1, α3, and α5 containing receptors co-expressed with the β2 and γ2 subunits ELB139 enhances the GABA-induced currents but was less potent and less efficient than diazepam (Table 1). 10 µM of Ro15-1788 completely abolished the effects of ELB139 in all of these three GABA_{A} receptor combinations. In α1-containing receptors positive current modulation by ELB139 was observable at 0.3 µM and reached its maximum of 1.6 ± 0.08-fold at 30 µM (Fig. 5A), which is about half of the maximal diazepam effect (2.2 ± 0.9-fold at 1 µM diazepam). In α5β2γ2 receptors a positive current modulation of ELB139 was first observed at 1 µM and reached at 30 µM its maximum of 1.4 ± 0.06-fold, i. e., the efficacy and potency of ELB139 were significantly lower than diazepam which potentiated the current at a dose of 10 nM and reached its maximal agonistic effect of 2 ± 0.05-fold current enhancement at 1 µM (Fig. 5E). The new compound ELB139 positively modulated GABA-induced currents in α3-containing receptors at concentrations above 30 nM with the highest efficacy values of 1.3 ± 0.07-fold at concentrations above 0.3 µM. In contrast, positive current modulation was observable at concentrations below 10 nM for diazepam and reached the maximal stimulation of 1.8 ± 0.11-fold at 1 µM.

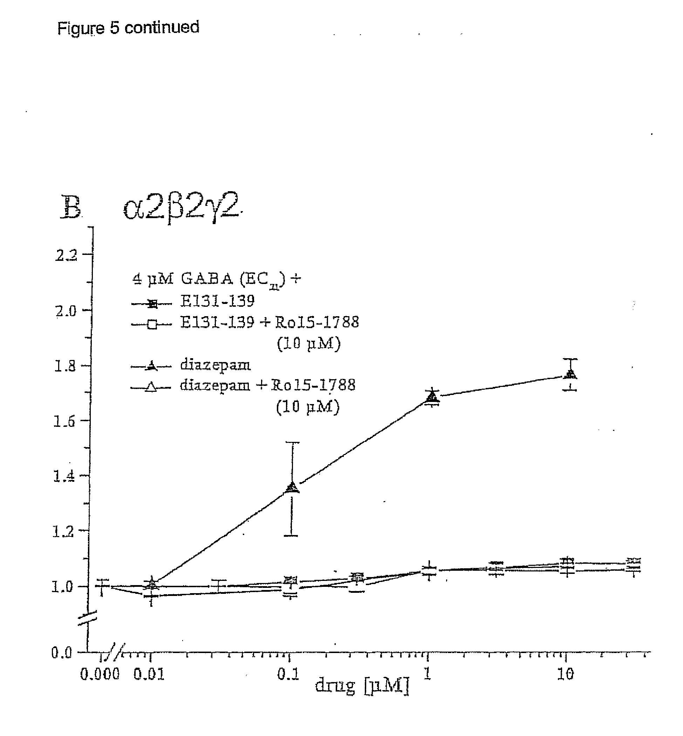

Compared to the prototypical BZ diazepam, ELB139 demonstrated an α subunit specificity on α2 containing receptors. On this GABA_{A} receptor, diazepam showed high efficacy and potency with positive current modulations first measurable at concentrations below 0.1 µM and maximal agonism at 1 with a potentiation of 1.8-fold (Fig. 5B, Table 1). In contrast, ELB139 failed to evoke agonistic or inverse agonistic modulation of the GABA-induced currents in recombinant α2β2γ2 GABA_{A} receptors.

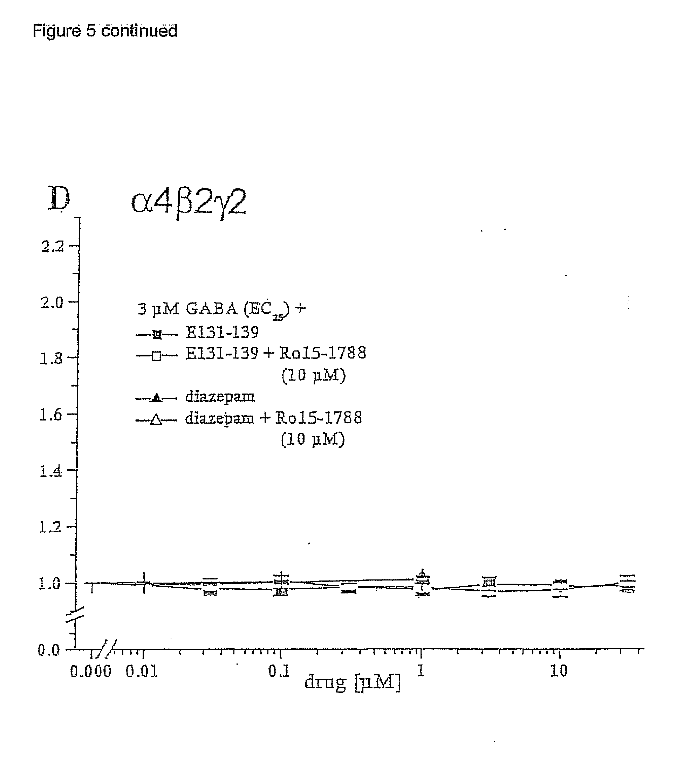

For α4-containing receptors diazepam displayed no significant modulation of the GABA induced currents up to concentrations of 1 µM (Fig. 5D). Likewise, the CI⁻ current was unaffected by ELB1 9 at all concentrations tested.

Whereas the potency of ELB139 in α1β2γ2 and α5β2γ2 receptors was similar, i.e., in both cases significant agonistic effects were noticeable at about 1 µM ELB139, the efficacy of this drug for α1-containing receptors was slightly higher than for α5β2γ2 receptors (1.6-fold versus 1.4, see Table 1). Interestingly, ELB139 showed the highest potency on α3β2γ2 receptors with potentiating effects seen already at concentrations above 30 nM, but it exhibited the lowest efficacy with only 1.3-fold current potentiation on these receptors.

**Table 1: EC₅₀ and potentiation values of ELB139 and diazepam applied together with the approximate receptor subtype specific GABA EC₂₀. Asterisks (*) indicate graphically determined values.**

| αiβ2γ2 | **ELB139** | | **Diazepam** | |
|---|---|---|---|---|
| | EC₅₀ [µM] | Potentiation* | EC₅₀* [µM] | Potentiation* |
| α1 | 3.8 | 1.6±0.08 | 0.035 | 2.2±0.9 |
| α2 | >>30 | -- | 0.1 | 1.8±0.6 |
| α3 | 0.06* | 1.3±0.07 | 0.09 | 1.8±0.11 |
| α4 | >>30 | -- | >>1 | -- |
| α5 | 3.6 | 1.4±0.06 | 0.07 | 2±0.05 |

### 4.5. Conclusion

In conclusion, the new drug ELB139 positively modulated GABA-induced currents on α1, α3, and α5β2γ2 GABA_{A} receptors via the BZ-binding site with lower potency and efficacy compared to diazepam. However, ELB139 provided about 50-fold selectivity for α3 containing GABA_{A} receptors versus α1 and α5. Furthermore, in contrast to diazepam, ELB139 showed no agonistic effect on α2-containing receptors. The results indicate that the divergent GABA_{A} receptor subtype selectivity of ELB139 as compared to diazepam might underlie the differences in their *in vivo* activities.

### Example 5

In vivo antidepressant activity of ELB139 as an example of a subtype selective partial agonistic alpha 3 preferring compound in a rodent model of depression

The effect of ELB139 was examined in the rat forced swim test (FST). Depressive disorders, including major depression, are serious and disabling. Selective serotonin reuptake inhibitors (SSRIs) have improved safety and tolerability of antidepressant treatment. However, compliance is often hampered by adverse drug effects, mainly during the initial phases of treatment. The antidepressant efficacy of the SSRIs, particularly in severely depressed patients, is not superior to that of tricyclic antidepressants (about 30% of the patients show no improvement) (Anderson and Thomenson, 1994; Blier, 2001 Anderson and Tomenson, 1994; Burke and Preskom, 1995). For this reason, there is considerable interest in new therapeutic approaches in the treatment of depression.

Approximately a dozen animal tests for antidepressant agents are used (Cryan et al., 2002 Cryan et al., 2002). The forced swim test, used to measure non-specific resistance to stress, was described and validated by Porsolt and co-workers (Porsolt et al., 1977) for the use in rats and in mice (Porsolt, 2000). The forced swim test exploits a behavioural approach in which the animal is forced to swim to nearly complete exhaustion (about 15 minutes). Following a period of forceful activity at the beginning, the rodent adopts quickly a typically immobile posture, moving only minimal to keep floating (Porsolt et al., 1977). The validity of the forced swim test (Porsolt test) and its relationship to depression have been reviewed extensively (Cryan et al., 2002; Willner, 1984; Willner, 1990) and it is an established screening test for antidepressant agents. Although different laboratories have made technical modifications in the apparatus, the fundamentals of the test remained the same. Unfortunately, the detection of selective serotonin reuptake inhibitors (SSRIs), although clinically effective, is unreliable in the conventional forced swim test (Borsini and Meli, 1988 ; Detke et al., 1995 ; Cryan, 2002). A modification of the FST, based on a detailed analysis of the behaviour allows the detection and discrimination of serotonergic and noradrenergic antidepressants more reliably (Lucki, 1997, Detke et al., 1995).

### 5.1. Material and Methods

### Animals

Male Wistar rats (Shoe: Wist, Dimed Schönwald GmbH, Germany) of 180-220 g body weight were used. They were group-housed, 5 per cage (45x60x25 cm), at room temperature (22 ± 2 °C) and with a 12 h light-dark cycle (light on at 06.00 hours) illuminated with 170 lux. Standard pellet food (Altromin 1326) and water were freely available. To ensure adaptation to the new environment the rats were housed in the departmental animal unit for two weeks before testing. The rats were assigned randomly to the treatment groups on arrival. The tests were performed in a sound proofed, brightly illuminated room between 14.00 and 17.00 hours.

### Chemicals

| | |
|---|---|
| Test compound: | ELB139 |
| Chemical name: | 1-(p-chlorphenyl)-4-piperidin-1-yl-1,5-dihydro- |
| | imidazo-2-on |
| Mol. Weight: | 277.75 |
| Batch: | S306767 |
| Manufacturer: | elbion AG |
| | |
| Reference compound : | fluoxetine |
| Chemical name: | N-methyl-γ-[4-(trifluoromethyl)phenoxy] |
| | benzenepropanamine |
| Mol. Weight: | 345.8 |
| | |
| Vehicle 1: | Tylose |
| Chemical name: | Hydroxyethylcellulose |
| Batch: | S22341 743 |
| Manufacturer: | Merck Eurolab GmbH |
| | |
| Vehicle 2 : | PEG 300 |
| Batch : | Lot53616433 |
| Manufacturer: | Merck Eurolab GmbH |

### Drug administration schedule/dosage

ELB139 (10, 30 mg/kg) was freshly suspended in 10% PEG + 90% 0.5% hydroxyethylcellulose prior the experiments. The established antidepressant, the SSRI fluoxetine (10, 30 mg/kg) and the vehicle treated controls (10% PEG + 90% 0.5% hydroxyethylcellulose) were included to validate the experimental procedures.

All animals were treated with verum or vehicle three times: shortly after the habituation session (23h), 5 and 1 h prior to testing. All drugs were administered orally with an application volume of 1 ml/kg.

### 5.2. Experimental procedure

The animals were tested in a glass tank (23 x 30 cm, height 40 cm) filled to a depth of 28 cm with water at 22°C (the animals could not touch the bottom). The glass tank was illuminated indirectly and was surrounded by dark brown shading walls (distance from the tank 20 cm) to prevent the view on the experimenter. The experiments were performed between 14:00 and 17:00 hours and the method was in general performed as described (Porsolt et al., 1979; Lucki, 1997).

On the first experimental day, rats were gently placed in the water for a 15 min period of habituation. On removal from the water, they were placed in a standard plexiglass box, the floor covered with paper towels, under an infrared heater for 30 min to dry. The next day, they were once more placed gently in the glass tank and observed for 5 min. The behaviour of the animals was video-taped.

At the end of the 5 min period, the rats were transferred to the infrared heated box and allowed to dry.

Following the experiment the video tapes were analysed manually and the duration of the following behaviours was recorded: Immobility: floating and making only those movements necessary to keep the nose above the water. Swimming: when an animal exhibits active motions, i.e. moving around the tank including diving. Climbing: when rats strongly move their forepaws in and out of the water, usually against the walls.

### 5.3. Statistics

Data are presented as mean ± S.E.M. and the group size was 10 rats. Comparisons between groups were carried out with a One Way ANOVA followed by intergroup comparisons using the Holm-Sidak method. Results were considered as significant for values of P<0.05. All statistical procedures were carried out using SigmaStat version 3.0. Individual data are shown in Table 2-4.

### 5.4. Results

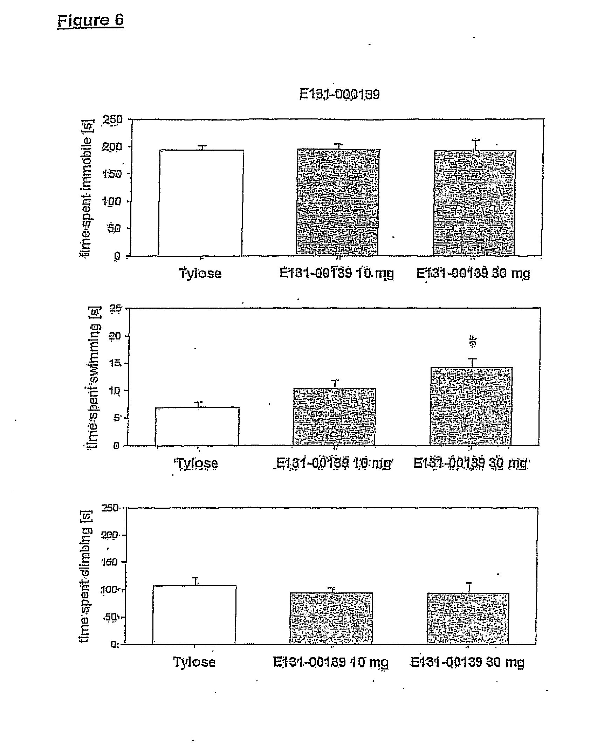

ELB139 had no effect on the time spent immobile during the forced swim test but increased the duration of swimming in the dose of 30 mg/kg p. o., while the climbing behaviour was not changed (Fig.6). Subjective observations suggest a moderate hypoactivity following the return to the homecage at 30 mg/kg p.o.

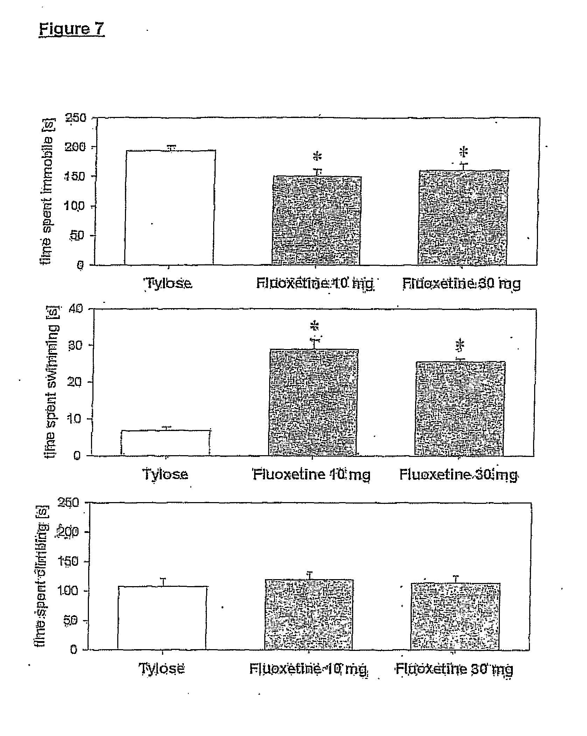

The established antidepressant, fluoxetine reduced the time spent immobile and increased the swimming time at 10 and 30 mg/kg, while the climbing behaviour was not changed (Fig. 7).

### 5.5. Discussion

In our study ELB139 showed an effect in the forced swim test. To enquire more information about the putative antidepressant-like effects, the behaviour during the forced swim test was analysed in the more complex method allowing the differentiation between various classes of antidepressant agents (Lopez-Rubalcava and Lucki, 2000; Lucki, 1997). Consistent with previous studies the selective serotonin reuptake inhibitor fluoxetine boosted swimming behaviour without increasing the climbing behaviour.

The effects of ELB139 on the measured parameters in the forced swim test are similar to those of fluoxetine (alteration in swimming while having less effect on the duration of the immobility), instead of those of desipramine (changes in immobility and climbing) (Rex et al., in press). Therefore, it is imaginable that the action of ELB139 may involve the serotonergic system. Decreased serotonergic neurotransmission has been proposed to play a key role in the aetiology of depression. This has been established mainly by the clinical efficacy of the third generation antidepressants, the SSRIs, which enhance serotonergic transmission (Beique et al., 2000; Blier, 2001). ELB139 was well tolerated at both doses of 10 and 30 mg/kg.

During the test session, the rats having received 10 or 30 mg/kg ELB139 did not show an increase of immobility time but at 30 mg/kg a slight but not significant decrease of climbing time could be detected. This may be due to a reducing effect of ELB139 on locomotor activity. There was also a slight reduction of the activity of the rats to be seen after returning them to the homecage. However, this decline in the climbing time goes along with a concurrent significant increase of swimming time so that the total activity of the rats seems to be constant. Thus, ELB139 did not show a significant reduction of locomotor activity in the open field test and in different animal models of anxiety (Langen, 2002; Langen, 2003a+b). Yet, in contrast to fluoxetine ELB139 did not increase total activity of the rats.

In summary, considering the significant increase in swimming behaviour at the dose of 30 mg/kg p. o., ELB139 can be considered as a candidate for antidepressive treatment.

### Example 6

Reversal of the effect on serotonine levels of ELB139 using the benzodiazepine antagonist flumazenil

### 6.1. Materials and Methods

### Animals

Male Wistar rats (Crl: (WI) BR, Charles River, Sulzfeld, Germany) weighing 200 to 260 g were used for the experiment. They were housed under standard conditions in groups of five on a 12 h light/dark cycle (light on at 0600 h) with ad libitum access to food (Pellets, ssniff M/R 15, Spezialdiat GmbH, Soest/Westfalen) and water.

### Chemicals

ELB139 (1-(p-chlorphenyl)-4-piperidin-1-yl-1,5-dihydro-imidazo-2-on, MW 277.75) was manufactured by elbion AG. Flumazenil (8-Fluoro-5-methyl-6-oxo-5,6-dihydro-4H-2,5,10b-triaza-benzo[e]azulene-3-carboxylicacidethylester) was obtained by Tocris, distributed by Biotrend Chemikalien GmbH, Köln, Germany. All other chemicals used were obtained from Sigma-Aldrich Chemie GmbH, Germany or from Merck, Germany.

### Drug administration schedule and dosage

Route of application: i. p.
Applied volume: 0.5 ml/100g

| substance | Dosage [mg/kg] | pre-treatment time [min] | number of application [n] |
|---|---|---|---|
| flumazenil | 10 | 40 min post ELB139 administration | 1 |
| ELB139 | 30 | 0 | 1 |

### 6. 2. Preparation of compounds

ELB139 was freshly suspended in 90% 0.5%-hydroxyethylcellulose and 10% PEG 300 so that an administration volume of 0.5 ml/100 g was reached for each substance and dose. Haloperidol injection solution was diluted with saline so that an administration volume of 0.5 ml/100 g was reached. Flumazenil was diluted with saline so that an administration volume of 0.5 ml/100 g was reached. The solution and the suspension were placed on a magnetic stirrer before and during dosing procedures. Hydroxyethylcellulose was dissolved in distilled water.

### 6.3. Experimental procedure

### Surgery

The day before the experiment, male rats were anaesthetised with chloral hydrate (3.6%, 1ml/100 g i.p.) and placed in a stereotaxic frame to implant a microdialysis guide cannula (CMA/12, Carnegie Medicine, Sweden). Scalp was incised in the median anterior-posterior direction between lambda and bregma and a small hole was drilled into the skull. The stereotaxic coordinates were AP = +1.0 mm, L = -3.0 mm from bregma and 1.5 mm from the skull surface for the striatum according to the atlas of Paxinos and Watson, (1986) (1). Dental cement (Sinfony (2)) was used to fix the guide cannula and anchor screws to the cranium. During the surgery rectal temperature was maintained at 37°C using a heating blanket.

### Microdialysis

The day before the experiment the microdialysis probe (CMA/12, membrane length 4mm, Carnegie Medicin, Sweden) was inserted into the striatum through the guide canula. On the day of experiment the probe was perfused with Ringer solution (148 mM NaCl, 4 mM KCI, 2,4 mM CaCl₂, pH = 6,0). The flow rate (1 µl/min) allowed to collect 20 µl samples every 20 minutes into microvials. Microvials were stored in a fraction collector (CMA/170) at a temperature of 8°C until analysed. Using a swivel joint the perfusion arrangement allowed the animal to move freely within a hemispherical bowl. After an adjusting time of one hour 3 consecutive 20-min-fractions were collected to establish a stable basal level of neurotransmitter release.

Thereafter 30 mg/kg ELB139 or an equivalent volume of 0.5 % tylose, respectively, was administered. Then collecting samples was continued for at least 220 minutes.

### Analysis of dialysates

Dialysates were directly analysed by reverse-phase high-performance liquid chromatography with electrochemical detection (HPLC-EC). The samples were separated by a ZORBAX SB-Aq 2.1 mm ID x 100 mm column (Agilent Technologies). 1 µl 1% perchloric acid were added to the 20-minutes-fraction and 10 µl of this mixture were injected into the HPLC-system.

The mobile phase contained:

| | |
|---|---|
| KH₂PO₄ | 50mM |
| Octan-1-sulfonsaure Natriumsalz | (NOS) 2, 2mM |
| EDTA | 0.086mM |
| 2MH₃PO₄ | 5ml |
| Methanol (MeOH) | 83ml |
| Acetonitril | 10ml |
| | at pH 3.5 |

The method (serotonin) runs at a flow of 0.23 ml/min and a column temperature of 38°C, sample thermostat 8°C.

At night flow was reduced to a flow of 0.1 ml/min. Catecholamines were oxidized at 500 mV (Model 5014 B microdialysis cell, esa). Dopac was measured at 200 nA, dopamine at 2 nA, HIAA at 100 nA, HVA at 2 nA, and serotonin at 500 pA.

In order to calibrate the system external standards with 5 concentrations each were run regularly:

| | |
|---|---|
| DOPAC (3, 4-dihydroxyphenylacetic acid) | 1000, 500, 250, 125, 62.5 nM |
| DOPAMINE (3-hydroxytyramine hydrochloride) | 8, 4, 2, 1, 0.5 nM |
| HIAA (5-hydroxy-3-indoleacetic acid) | 200, 100, 50, 25, 12.5 nM |
| HVA (homovanillic acid) | 800, 400, 200, 100, 50 nM |
| SEROTONIN (5-hydroxytryptamine-kreatininsulfat) | 0.15, 0.075, 0. 0375, 0.01875, |
| | 0.009375 nM |

Before implanting and after removing the microdialysis probe a recovery was carried out with a solution containing 2000 nM DOPAC, 40 nM dopamine, 1000 nM HIAA, 2000 nM HVA and 0.8 nM serotonin. The recovery rate of the probes lay between 5 and 20 %.

### Histology

After completion of the experiment the brain of the rats was removed and postfixed in formalin (10%) for approximately 10 days. The brains were cut using vibroslice (TSE) and stained with toloidin blue to prove probes right position.

### 6. 4. Statistics

Basal level of 5-HT release showed interindividual differences. Therefore data of each animal were expressed as percentages. Data from dialysates before administering the substances were averaged, and the mean set as 100 %; all individual values were calculated accordingly.

Results were analysed by two way analysis of variance (ANOVA) with time and drug as the two factors. Tuckey test was used for individual comparison. P < 0.05 was regarded as significant.

### 6.5. Results

ELB139 induces a marked increase of extracellular serotonin in the striatum of rats compared to mean basal level. When giving flumazenil at 10 mg/kg i.p. 40 minutes after the administration of ELB139 at 30 mg/kg i. p. the increase of extracellular serotonin is not only reversed but serotonin level even decreases below the mean basal level. At the end of the recording time (3 h) serotonin level returns to mean basal level again.

### Example 7

Reversal of the anti-psychotic effect of ELB139 using the benzodiazepine antagonist flumazenil

### 7.1. Materials and Methods

### Animals

Female Wistar rats (Crl : (WI) BR, Charles River, Sulzfeld, Germany) weighing 168 to 217 g were used for the experiment. They were housed under standard conditions in groups of five on a 12 h light/dark cycle (light on at 0600 h) with ad libitum access to food (Pellets, ssniff M/R 15, Spezialdiät GmbH, Soest/Westfalen) and water.

### Chemicals

ELB139 (1-(p-chlorphenyl)-4-piperidin-1-yl-1,5-dihydro-imidazo-2-on, MW 277.75) was manufactured by elbion AG. Haloperidol (4-(4-[4-chlorophenyl]-4-hydroxy-1-piperidinyl)-1-(4-fluorophenyl)-1-nutanone, MW 375,9) was obtained by ratiopharm GmbH, Ulm, Germany, MK-801 (dizocilpine, MW 337.37) and flumazenil (8-Fluoro-5-methyl-6-oxo-5,6-dihydro-4H-2,5,10b-triaza-benzo[e]azulene-3-carboxylicacidethylester) were obtained by Tocris, distributed by Biotrend Chemikalien GmbH, Köln, Germany. All other chemicals used were obtained from Sigma-Aldrich Chemie GmbH, Germany or from Merck, Germany.

### 7. 2. Drug administration schedule and dosage

Applied volume: 0.5 ml/100g

| substance | Number of animals | Dosage [mg/kg] | pre-treatment | number of application | Route of administration |
|---|---|---|---|---|---|
| | [n] | | [min] | [n] | |
| MK-801 | 6 | 0. 1 | 10 | 1 | i.p. |
| ELB139 | 6 | 30 | 60 | 1 | p.o. |
| ELB139 + flumazenil | 6 | 30 5 | 60 20 | 1 1 | p.o . i.p. |
| haloperidol | 9 | 0.5 | 30 | 1 | i.p. |
| haloperidol + flumazenil | 4 | 0.5 5 | 60 20 | 1 1 | i.p. i.p. |

### 7.3. Preparation of compounds:

ELB139 was freshly suspended in 90% 0.5%-hydroxyethylcellulose and 10% PEG 300 so that an administration volume of 0.5 ml/100 g was reached for each substance and dose. Haloperidol injection solution was diluted with saline so that an administration volume of 0.5 mol/100 g was reached. Flumazenil and MK-801 were diluted with saline so that an administration volume of 0.5 ml/100 g was reached. The solutions and the suspension were placed on a magnetic stirrer before and during dosing procedures. Hydroxyethylcellulose was solved in distilled water.

Apart from the haloperidol rats which received haloperidol 30 minutes and MK-801 10 minutes prior to the test, the other test groups received haloperidol, ELB139 or vehicle 60 minutes prior to test, saline or flumazenil 20 minutes prior to test and MK-801 10 minutes prior to test.

### 7.4. Experimental procedure

The behaviour induced by the NMDA antagonist MK-801 is generally accepted as a rat model of psychosis. MK-801 induces stereotypies, hyperactivity and ataxia in rats after intraperitoneal administration.

Locomotor activity of the rats is recorded by the MotiTest Apparatus (TSE, Bad Homburg, Germany). The test area consisted of a squared arena (45 x 45 cm) with protective plexiglass walls (20 cm of height) where rats could freely move. Horizontal movements were recorded by 32 infrared photocells arranged along the bottom of each wall of the arena. Vertical movements (rearings) were recorded by a horizontal row of 32 infrared photocells 12 cm above the floor. The following parameters were measured by the computer program "ActiMot" (TSE, Bad Homburg, Germany):
1. active time [s]
2. total distance travelled [m]

Stereotypies, divided into stereotyped sniffing and other stereotypies, and ataxia are scored by the experimenter every five minutes for one hour (12 intervals) according to the method described by Andiné et al. (1999). The scores of the 12 intervals are added for each parameter.

| score | stereotyped sniffing | other stereotypies | ataxia |
|---|---|---|---|
| 0 | no stereotyped sniffing | no other stereotypies | normal body control |
| 1 | discontinuous sniffing (free interval > 5 s) | discontinuous stereotypies (free interval > 5 s) | fatting tendency upon movement |
| 2 | continuous sniffing | continuous stereotypies | falling upon movement |
| 3 | - | - | almost unable to move |

The day of experiment the female rats are placed in the laboratory and receive the test compound, the reference substance or vehicle at the appropriate time prior to test. MK-801 0.2 mg/kg is intraperitoneally administered 10 minutes prior to test.

At the beginning of the test the rats are placed in the centre of the squared arena of the MotiTest apparatus. Behaviour of the rats is recorded for one hour. After each run animals are removed and the boxes thoroughly cleaned and dried.

### 7.5. Statistics

Results of sniffing, other stereotypies and ataxia were analysed by one way analysis of variance (ANOVA). Tukey test was used for individual comparison. Results of activity and total distance travelled were analysed by two way analysis of variance (ANOVA) (compound x time). Student-Newman-Keuls test was used for individual comparison. P < 0.05 was regarded as significant.

### 7.6. Results

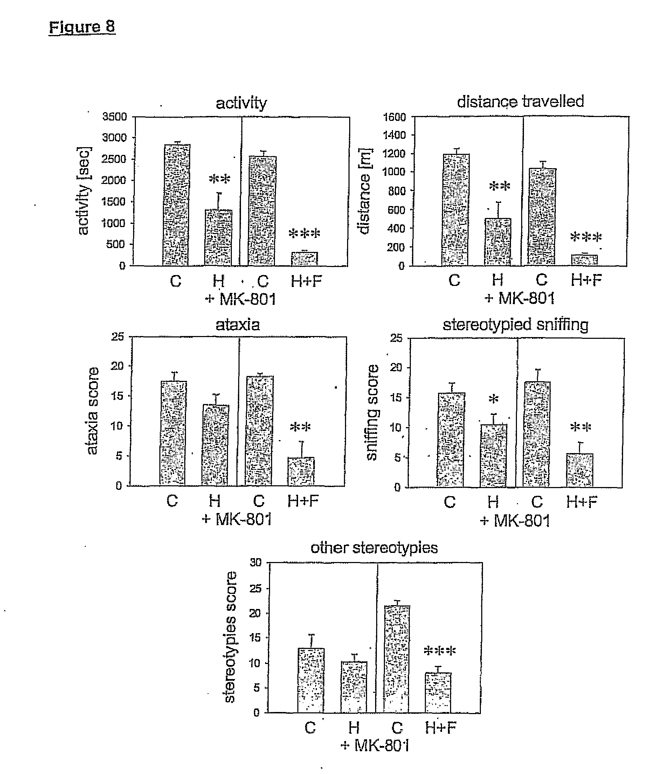

Haloperidol at 0.5 mg/kg i. p. significantly reversed the increased activity, the increased distance travelled and the sniffing (stereotypies) induced by MK-801 (figure 1). The other stereotypies induced by MK-801 were slightly reduced by haloperidol at 0.5 mg/kg. These effects of haloperidol were not affected by flumazenil at 5 mg/kg. The MIK-801-induced ataxia was only marginally reduced by haloperidol but was significantly reversed by the administration of the combination of haloperidol and flumazenil (Figure 8).

The data of ELB139 were gained in two separated trials performed by two different laboratory assistants (Nov 2003 and Jan 2004). The two separated trials and the sum of both trials are described and shown in Figures 9 and 10a and b.

At 30 mg/kg p.o. ELB139 significantly reversed the stereotyped sniffing in the discrete trials and when adding up the data of both trials. The effect of ELB139 was distinctly antagonised by flumazenil in the second trial and significantly reversed in the first trial and in the sum of both trials (Figure 9).

The other stereotypies induced by MK-801 were slightly reduced in the first and significantly reversed in the second trial and when summing up the data of both trials by ELB139 at 30 mg/kg p. o.. This effect of ELB139 was rather amplified by flumazenil at 5 mg/kg in the first trial and distinctly reversed in the second trial so that when adding the data no change of the ELB139 effect by flumazenil was to be seen anymore. These controversial results were probably caused by an imprecise definition of "other stereotypies" in the first trial (Figure 9).

At 30 mg/kg p. o. ELB139 hardly affected the ataxia induced by MK-801 in the first trial (Nov 2003). In the second trial (Jan 2004) this effect was more pronounced but still not significant so that, when summing up both trials, only a slight reduction of ataxia was to be seen. The reducing effect of ELB139 on ataxia seen in the second trial was reversed by flumazenil at 5mg /kg i. p.. When summing the data of both trials the reversing effect of flumazenil was marginal (Figure 9).

MK-801-induced hyperactivity was recorded as activity and distance travelled and described in 5-minute-intervalls. When considering the whole time curves ELB139 at 30 mg/kg p. o. distinctly reduced the activity induced by MK-801 in the second trial and significantly reduced it in the first trial and when summing the data of both tests (two way ANOVA, Figure 10a). In both tests and in the sum of both tests the effect of ELB139 on activity was significantly reversed by flumazenil at 5 mg/kg i.p. (Figure 10a).

The MK-801 induced increase of the distance travelled was significantly reduced by ELB139 in both tests and the sum of both tests when contemplating the whole time curve (two way AnOVA, Figure 10a). This effect was significantly reversed by flumazenil in all trials and when summing up the data of both trials (Figure 10a).

When contemplating the single time points the effect of ELB139 on activity and distance travelled was more pronounced during the first half of an hour (Figure 10b).

### Aspects of the invention

### Aspects of the invention are:

- A use of an effective amount of at least one 1-ar(alk)ylimidazolin-2-one of formula (I) 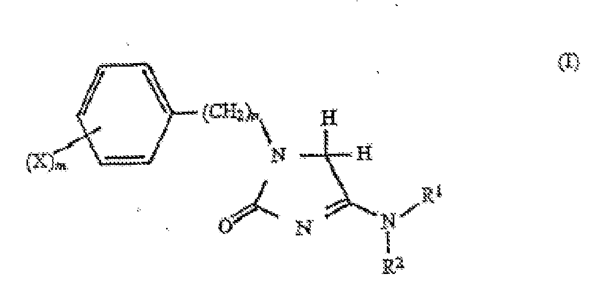 in which X is hydrogen, a C₁₋₄-alkyl, C₁₋₄ alkoxy, trifluoromethyl, or a halogen residue, R¹ and R² are independently of each other a C₁₋₄-alkyl, C₃₋₁₀ cycloalkyl or C₃₋₁₀ heteroalkyl residue, or R¹ and R² are together a C₂₋₆ akylene residue in which a -CH₂-group is optionally replaced by oxygen, nitrogen or sulfur, n is 0 or 1, and m is 0 or a cardinal number from 1 to 5 for the manufacture of a medicament for treating or preventing central nervous system disorders including psychotic disorders, movements disorders and/or psychotic symptoms associated to other mental disorders by administering to a patient in need thereof;
- a use of an effective amount of at least one substance which is a subtype selective agonist of benzodiazepin receptors carrying the alpha 3 subunit but is not active on receptors carrying the alpha 2 or alpha 4 subunit of the GABA_{A} receptor for the manufacture of a medicament for treating or preventing central nervous system disorders including psychotic disorders, movements disorders and/or psychotic symptoms associated to other mental disorders and especially for treating anxiety disorders.
- a use according to this aspect of the invention, wherein the substance acts partially agonistic with high affinity on alpha 3 carrying receptors and in addition, with low affinity, partially agonistic on alpha 1 and/or alpha 5 carrying receptors;
- a use according to the invention, wherein 1-(4-chlorphenyl)-4-piperidinoimidazolin-2-one is used;
- a use according to the invention, wherein the compound of formula (I) is formulated for parenteral or oral administration;
- a use according to the invention, wherein the compound of formula (I) is provided for administration in an amount of 1-100 mg/kg of the body weight of the patient;
- a use according to the invention, wherein the central nervous system disorder is a psychosis or psychotic episode;
- a use according to the invention, wherein the psychosis or psychotic episode is a type of schizophrenia (for instance paranoid, disorganized, catatonic undifferentiated or residual), a bipoar mood disorder, such as manic depression, and the post-psychotic depressive disorders of schizophrenia. Psychotic episodes to be seen with schizophreniform disorders, schizoaffective disorders, delusional disorders, substance-induced psychotic disorders (for instance induced by alcohol, amphetamine, cannabis, cocaine, halluzinogens, inhalants, opioids, or phencyclidine); personality disorders (such as borderline personality disorder) impulsive disorders, such as maladaptive aggression; bipolar disorders and hyperactive-impulsive attention deficit/hyperactivity (AD/HD) and abuse and addiction (for example alcohol, amphetamine, cocaine or opiate addiction);
- a use according to the invention, wherein the central nervous system disorder is a mood disorder or mood episode;
- a use according to the invention, wherein the mood disorder or mood episode is a major depressive disorder or episode, manic, mixed and hypomanic mood episodes, depressive episodes with atypical, catatonic or melancholic features, depressive episodes with postpartum onset premenstrual dysphoric disorder, minor depressive disorder, post traumatic, acute stress disorder, obsessive-compulsive disorder and patients with eating disorders;
- a use according to the invention, wherein the central nervous system disorder is an anxiety disorder or episode of anxiety;
- a use according to the invention, wherein the anxiety disorder or episode is chronic anxiety disorder, panic disorder, agoraphobia, specific phobia, social phobia and generalized anxiety disorder;
- a use according to the invention, wherein the central nervous system disorder is a movement disorder which is primarily associated to malfunction of basal ganglia;
- a use according to the invention, wherein the movement disorder is different subtypes of dystonia, such as focal dystonias, multiple-focal or segmental dystonias, torsion dystonia, hemispheric, generalised and tardive dystonias (induced by psychopharma-cological drugs). Focal dystonias include cervical dystonia (torticolli), blepharospasm (cramp of the eyelid), appendicular dystonia (cramp in the extremities, like the writer's cramp), oromandibular dystonia and spasmodic dysphonia (cramp of the vocal cord) and paroxysmal dystonia;
- a pharmaceutical composition for the treatment or prevention of central nervous system disorders including psychotic disorders, movement disorders and/or psychotic symptoms associated to other mental disorders containing an effective amount of at least one 1-ar(alk)ylimidazolin-2-one of formula (I) 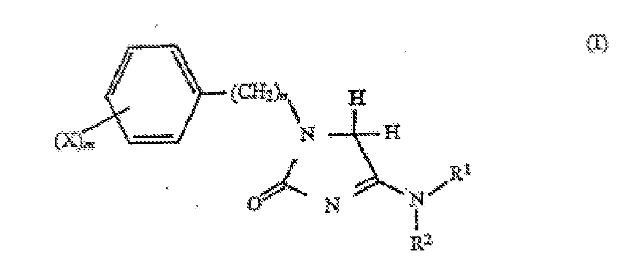 in which X is hydrogen, a C₁₋₄-alkyl, C₁₋₄ alkoxy, trifluoromethyl, or a halogen residue, R¹ and R² are independently of each other a C₁₋₄-alkyl, C₃₋₁₀ cycloalkyl or C₃₋₁₀ heteroalkyl residue, or R¹ and R² are together a C₂₋₆ akylene residue in which a -CH₂-group is optionally replaced by oxygen, nitrogen or sulfur, n is 0 or 1, and m is 0 or a cardinal number from 1 to 5 to a patient in need thereof;
- a pharmaceutical composition for the treatment of central nervous system disorders including psychotic disorders, movement disorders and/or psychotic symptoms associated to other mental disorders containing a benzodiazepin receptor ligand which is selective for the alpha 3 subunit of the benzodiazepin receptor but does not exert a significant positive GABA increasing effect on receptors carrying the alpha 2 and/or alpha 4 subunit of the GABA receptor;
- a pharmaceutical composition according to this aspect of the invention according to claim 16, wherein the benzodiazepine ligand acts partially agonistic with high affinity on alpha 3 carrying receptors and in addition, with low affinity, partially agonistic on alpha 1and/or alpha 5 carrying receptors;
- a pharmaceutical composition according to the invention, wherein the compound of formula (I) is 1-(4-chlorphenyl)-4-piperidinoimidazolin-2-one;
- a pharmaceutical composition according to the invention, wherein it contains further excpients or auxiliaries;
- a pharmaceutical composition according to the invention for parenteral or oral administration;
- a pharmaceutical composition according to the invention, wherein it contains 1-100 mg/kg body weight of the patient of the compound of formula (I);
- a pharmaceutical composition according to the invention, wherein the central nervous system disorder is a psychosis or psychotic episode;
- a pharmaceutical composition according to the invention, wherein the psychosis or psychotic episode is a type of schizophrenia (for instance paranoid, disorganized, catatonic undifferentiated or residual), a bipoar mood disorder, such as manic depression, and the post-psychotic depressive disorders of schizophrenia. Psychotic episodes to be seen with schizophreniform disorders, schizoaffective disorders, delusional disorders, substance-induced psychotic disorders (for instance induced by alcohol, amphetamine, cannabis, cocaine, halluzinogens, inhalants, opioids, or phencyclidine); personality disorders (such as borderline personality disorder) impulsive disorders, such as maladaptive aggression; bipolar disorders and hyperactive-impulsive attention deficit/hyperactivity (AD/HD) and abuse and addiction (for example alcohol, amphetamine, cocaine or opiate addiction);
- a pharmaceutical composition according to the invention, wherein the central nervous system disorder is a mood disorder or mood episode;
- a pharmaceutical composition according to the invention, wherein the mood disorder or mood episode is a major depressive disorder or episode, manic, mixed and hypomanic mood episodes, depressive episodes with atypical, catatonic or melancholic features, depressive episodes with postpartum onset premenstrual dysphoric disorder, minor depressive disorder, post traumatic, acute stress disorder, obsessive-compulsive disorder and patients with eating disorders;
- a pharmaceutical composition according to the invention, wherein the central nervous system disorder is an anxiety disorder or episode of anxiety;
- a pharmaceutical composition according to the invention, wherein the anxiety disorder or episode is chronic anxiety disorder, panic disorder, agoraphobia, specific phobia, social phobia and generalized anxiety disorder;
- a pharmaceutical composition according to the invention, wherein the central nervous system disorder is a movement disorder which is primarily associated to malfunction of basal ganglia;
- a pharmaceutical composition according to the invention, wherein the movement disorder is different subtypes of dystonia, such as focal dystonias, multiple-focal or segmental dystonias, torsion dystonia, hemispheric, generalised and tardive dystonias (induced by psychopharmacological drugs). Focal dystonias include cervical dystonia (torticolli), blepharospasm (cramp of the eyelid), appendicular dystonia (cramp in the extremities, like the writer's cramp), oromandibular dystonia and spasmodic dysphonia (cramp of the vocal cord) and paroxysmal dystonia.

### References

Abi-Saab WM, D'Souza DC, Moghaddam B, and Krystal JH (1998). The NMDA anatgonist model for schizophrenia: promise and pitfalls. Pharmacopsychiatry 31 Suppl 2: 104 109.
American Psychiatric Association (1994). Diagnostic and Statistical Manual of Mental Disorders, 4th edn. American Psychiatric Press, Washington, DC.
Anderson IM, Tomenson BM. The efficacy of selective serotonin re-uptake inhibitors in depression: a meta-analysis of studies against tricyclic antidepressants. J Psychopharmacol 1994; 4: 238-249
Andiné P, Widermark N, Axelsson R, Nyberg G, Olofsson U, Martensson E, Sandberg M (1999). Characterization of MK-801-induced Behavior as a putative rat model of psychosis. J Pharmacol Exp Ther 290: 1393-1408.
Arnold, SE (1999). Neurodevelopmental abnormalities in schizophrenia: insights from neuropathology. Dev Psychopathol 11: 439-56.
Atack JR (2003). Anxioselective compounds acting at the GABA(A) receptor benzodiazepine binding site. Curr Drug Target CNS Neurol Disord 2: 213- 232.
Bailer J, Thurm-Mussgay I, Rey ER (1999). Schizophrenie. In: H. Reinecker (Hrsg.), Fallbuch der klinischen Psychologie, Kap 20, Göttingen: Hogrefe-Verlag: 343-66.
Beique J, Blier P, Debonnel G. Effects of sustained administration of the serotonin and norepinephrine reuptake inhibitor venlafaxine: I. In vivo electrophysiological studies in the rat. Neuropharmacology 2000; 39: 1800- 1812
Benavides, J. , Peny, B., Durand, A., Arbilla, S. , and Scatton, B. (1992). Comparative in vivo and in vitro regional selectivity of central w (benzodiazepine) site ligands in inhibiting [3H]flumazenil binding in the rat central nervous system. J Pharmacol Exp Ther 263, 884-896.
Benes, FM (1993). The relationship between structural brain imaging and histopathologic findings in schizophrenia research. Harv Rev Psychiatry 1: 100-109.
Blier P. Pharmacology of rapid-onset antidepressant treatment strategies. J Clin Psychiat 2001; 62 Suppl 15: 12-17
Atack JR (2003). Anxioselective compounds acting at the GABA (A) receptor benzodiazepine binding site. Curr Drug Target CNS Neurol Disord 2: 213-232.
Borsini F, Meli A. Is the forced swimming test a suitable model for revealing antidepressant activity Psychopharmacology 1988; 94: 147-60
Capuano B, Crosby IT, Lloyd EJ (2002). Schizophrenia: genesis, receptorology and current therapeutics. Curr Med Chem 9: 521-48.
Chambers MS, Collins IJ, Goodacre SC, Hallett DJ, Jones P, Keown LE, Maxey RJ, Street LJ (14.09.2001). World patent no. W02001 044249. New triazolopyrimidine compounds active as GABAA receptor ligands, useful for treating e. g. anxiety, neuroses, convulsions, migraine, depression, psychotic disorders, neurodegeneration pain, emesis or eating disorders.
Costa E and Guidotti A (1996). Benzodiazepines on trial: a research strategy for their rehabilitation. Trends Pharmacol Sci 17: 192-200.
Crestani F, Martin JR, Mohler H, Rudolph U (2000). Mechanism of action of the hypnotic zolpidem in vivo. Br J Pharmacol 131: 1251-1254.
Cryan JF, Markou A, Lucki I. Assessing antidepressant activity in rodents: recent developments and future needs. Trends Pharmacol Sci 2002; 23: 238-245
Davidson GC, Neale JM (1988) Klinische Psychologie, Kap. 14, München: Psychologische Verlags Union: 429-74.
Dawson, GR (23.01. 1998). World paten no. W09937370. Product for treating e. g. disorders of the central nervous system.
DDG eV = Deutsche Dystoniegesellschaft eV
Delgado P, Moreno F (1999). Antidepressants and the brain. Int Clin Psychopharmacol 14 Suppl 1: S9-S16.
Detke MJ, Rickels M, Lucki I. Active behaviors in the rat forced swimming test differentially produced by serotonergic and noradrenergic antidepressants. Psychopharmacology 1995; 121: 66-72
Dressler D, Munchau A, Bhatia KP, Quinn NP, Bigalke H (2002). Antibody-induced botulinum toxin therapy failure: can it be overcome by increased botulinum toxin doses? Eux Neuron 47: 118-21.
Dubinsky B, Vaidya AH, Rosenthal DI, Hochman C, Crooke JJ, DeLuca S, DeVine A, Cheo-Isaacs CT, Carter AR, Jordan AD, Reitz AB, Shank RP (2002). 5-ethoxymethyl-7-fluoro-3-oxo-1,2,3,5-tetrahydrobenzo[4,5]imidazo[1,2a]pyridine-4-N-(2-fluorophenyl) carboxamide (RWJ-51204), a new nonbenzodiazepine anxiolytic. J Pharmacol Exp Ther 303: 777-790.
Fahn S (1987). Systemic therapy of dystonia. Can J Neurol Sci 14: 528-32.
Fredow G, Loscher W (1991). Effects of pharmacological manipulation of GABAergic neurotransmission in a new mutant hamster model of paroxysmal dystonia. Eur J Pharmacol 192: 207-219.
Friedman J, Standaert DG (2001). Dystonia and its disorders. Neurol Clin 19: 681-705.
Gao B, Fritschy JM, Benke D, Mohler H (1993). Neuron-specific expression of GABAA-receptor subtypes: differential association of the alpha 1-and alpha 3-subunits with serotonergic and GABAergic neurons. Neuroscience 54: 881-892.
Gerlach J (2002). Life is not so easy... Psychopharmacology 162: 1-2.
Gernert M, Bennay M, Fedrowitz M, Rehders JH, Richter A (2002). Altered discharge pattern of basal ganglia output neurons in an animal model of idiopathic dystonia. J Neurosci 22: 7244-53.
Goodacre SC, Hallett DJ, Street LJ (07.02.2002). World patent no. W02002010170. New imidazo.pyrazine derivatives are useful as ligands for GABA receptors in treatment of e. g. anxiety, convulsion, panic disorder, social phobias, obsessive compulsive disorder, anxiety, migraine and schizophrenia.
Graeff FG, Guimaraes FS, DeAndrade TG, Deakin JFW (1996). Role of 5-HT in stress, anxiety and depression. Pharmacol Biochem Behav 54: 129-141.
Greene P (1992). Baclofen in the treatment of dystonia. Clin Neuropharmacol 15: 276-88.
Guyon A, Laurent S, Paupardin-Tritsch D, Rossier J, Eugene D (1999). Incremental conductance levels of GABAA receptors in dopaminergic neurones of the rat substantia nigra pars compacta. J Physiol 516: 719-737.
Hamann M, Richter A (2002). Effects of striatal injections of GABAA receptor agonists and antagonists in a genetic animal model of paroxysmal dystonia. Eur J Pharmacol 443: 59-70.
Hamill, O. P., Marty, A., Neher, E., Sakmann, B., and Sigworth, F. J. (1981). Improved patch-clamp techniques for high-resolution current recording from cells and cell-free membrane patches. Pflügers Arch 391, 85-100.
Herrero MT, Barcia C, Navarro JM (2002). Functional anatomy of thalamus and basal ganglia. Childs Nerv Syst 18: 386-404.
Hevers, W., and Luddens, H. (1998). The diversity of GABAA receptors. Pharmacological and electrophysiological properties of GABAA channel subtypes. Mol Neurobiol 18, 35-86.
Hsiung GY, Das SK, Ranawaya R, Lafontaine AL, Suchowersky O (2002). Long-term efficacy of botulinum toxin A in treatment of various movement disorders over a 10-year period. Mov Disord 17: 1288-93.
Jentsch JD, Roth RH (1999). The neuropsychopharmacology of phencyclidine: from NMDA receptor hypofunction to the dopamine hypothesis of schizophrenia. Neuropsychopharmacology 20: 201-25.
Jones N, King SM, Duxon MS (2002). Further evidence for the predictive validity of the unstable elevated exposed plus-maze, a behavioural model of extreme anxiety in rats: differential effects of fluoxetine and chlordiazepoxide. Behav Pharmacol 13: 525-535.
Kawata Y, Okada M, Murakami T, Kamata A, Zhu G, Kaneko S (2001). Pharmacological discrimination between effects of carbamazepine on hippocampal basal, Ca(2+)- and K(+)-evoked serotonin release. Br J Pharmacol 133: 557-567.
Ktein C, Ozeiius U (2002). Dystonia: clinical features, genetics, and treatment. Curr Opin Neurol 15: 491-7.
Korpi, E. R., and Lodens, H. (1993). Regional γ-aminobutyric acid sensitivity of t-butylbicyclophosphoro[35S]thionate binding depends on γ-aminobutyric acidA receptor α subunit. Mol Pharmacol 44, 87-92.
Krack P, Vercueil L (2001). Review of the functional surgical treatment of dystonia. Eur J Neurol 8 : 389-99.
Lahti AC, Weiler MA, Tamara Michaelidis BA, Parwani A, and Tamminga CA (2001). Effects of ketamine in normal and schizophrenic volunteers. Neuropsychopharmacology 25: 455-467.
Langen B (2002). Effect of AWD 131-139, 131-175 and 34-117 on anxiety-related behaviour in different animal models of anxiety. elbion AG, Pharmacological Research, Study report no.: E131-00139/PH-16/02, dated 2002-Oct-17
Langen B (2003a). Effect of E131-00139 on anxiety-related behaviour in two rodent models of anxiety. elbion AG, Pharmacological Research, Study report no.: E131-00139/PH-27/03, dated 2003-Sep-15
Langen B (2003b). Effect of flumazenil on the anxiolytic activity of E131-00139 in the elevated plus maze test in rats. elbion AG, Pharmacological Research, Study report no.: E131-00139/PH-28/03, dated 2003-Sep-15
Li XM, Perry KW, Fuller RW (1996). On the in-vivo modulation of neostriatal dopamine release by fluoxetine and 5-hydroxy-L-tryptophan in conscious rats. J Pharm Pharmacol 48: 825-828.
Lindenmayer JP, Czobor P, Volavka J, Lieberman JA, Citrome L, Sheitman B, Chakos, M, McEvoy JP (2002). Olanzapine in refractory schizophrenia after failure of typical or atypical antipsychotic treatment : an open-label switch study. J Clin Psychiatry 63: 931-5
Lopez-Rubatcava C, Lucki I. Strain differences in the behavioral effects of antidepressant drugs in the rat forced swimming test. Neuropsychopharmacology 2000; 22: 191-199
Low K, Crestani F, Keist R, Benke D, Brunig I, Benson JA, Fritschy JM, Rulicle T, Bluethmann H, Mohler H, Rudolph U (2000). Molecular and neuronal substrate for the selective attenuation of anxiety. Science 290: 131-134.
Lucki I. The forced swimming test as a model for core and component behavioral effects of antidepressant drugs. Behav Pharmacol 1997; 8: 523- 532
Lüddens, H., and Korpi, E. R. (1995). GABA antagonists differentiate between recombinant GABAA/benzodiazepine receptor subtypes. J Neurosci 15, 6957-6962.
Lüddens, H, Korpi, E. R., and Seeburg, P. H. (1995). GABAA/benzodiazepine receptor heterogeneity: neurophysiological implications. Neuropharmacol 34, 245-254.
McKernan R (30.09.1998). Great Britain patent no. GB2342043. Use of triazolopyridazine derivatives for the treatment of hearing loss, especially age-related hearing impairement and tinnitus.
Murakami T, Okada M, Kawata Y, Zhu G, Kamata A, Kaneko S (2001). Determination of effects of antiepileptic drugs on SNAREs-mediated hippocampal monoamine release using in vivo microdialysis. Br J Pharmacol 134: 507-520.
Nutt DJ, Forshall S, Bell C, Rich A, Sandford J, Mash J, Argyropoulos S (1999). Mechanism of action of selective serotonin reuptake inhibitors in the treatment of psychiatric disorders. Eur Neuropsychopharmacol 9 Suppl 3: S81-S86.
Nyberg S, Olsson H, Nilsson U, Maehlum E, Halldin C, Farde L (2002). Low striatal and extra-striatal D2 receptor occupancy during treatment with the atypical antipsychotic sertindole. Psychopharmacology 162: 37-41.
Okada M, Kawata Y, Kiryu K, Mizuno K, Wada K, Inomata H, Tasaki H, Kaneko S (1997). Effects of non-toxic and toxic concentrations of phenytoin on monoamines levels in rat brain. Epilepsy Res 28: 155-163.
Owens MJ, Nemeroff CB (1994). Role of serotonin in the pathophysiology of depression: focus on the serotonin transporter. Clin Chem 40: 288-295.
Pauls DL, Korczyn AD (1990). Complex segregation analysis of dystonia pedigrees suggests autosomal dominant inheritance. Neurology 40: 1107-10.
Paxinos G and Watson C (1986). The rat brain in stereotaxic coordinates. 2 nd ed. Academic Press Inc. London.
Pei Q, Zetterstrom T, Fillenz M (1989). Both systemic and local administration of benzodiazepine agonists inhibit the in vivo release of 5-HT from ventral hippocampus. Neuropharmaeology 28: 1061-1066.
Platt DM, Rowlett JK, Spealman RD, Cook J, Ma C (2002). Selective antagonism of the ataxic effects of zolpidem and triazolam by the GABAA/alpha1-preferring antagonist beta-CCt in squirrel monkeys. Psychopharmacology (Berl) 164: 151-159.
Porsolt RD, Bertin A, Blavet N, Deniel M, Jalfre M. Immobility induced by forced swimming in rats: effects of agents which modify central catecholamine and serotonin activity. Eur J Pharmacol 1979; 57: 201-210
Porsolt RD, Le Pichon M, Jalfre AA. Depression: a new animal model sensitive to antidepressant treatments. Nature 1977; 266: 730-732
Porsolt RD. Animal models of depression: utility for transgenic research. Rev Neurosci 2000; 11: 53-58
Prasad S, Semwal P, Deshpande S, Bhatia T, Nimgaonkar VL, Thelma BK (2002). Molecular genetics of schizophrenia: past, present and future. J Biosci 27: 35-52.
Pritchett, D. B. , and Seeburg, P. H. (1990). γ-aminobutyric acidA receptor α5-subunit creates novel type II benzodiazepine receptor pharmacology. J Neurochem 54, 1802-1804.
Pritchett, D. B., Lüddens, H. , and Seeburg, P. H. (1989). Type I and type II GABAA-benzodiazepine receptors produced in transfected cells. Science 245, 1389-1392.
Pritchett, D. B., Sontheimer, H., Shivers, B. D., Ymer, S., Kettenmann, H., Schofield, P. R., and Seeburg, P. H. (1989). Importance of a novel GABAA receptor subunit for benzodiazepine pharmacology. Nature 338, 582-585.
Rawicki B (1999). Treatment of cerebral origin spasticity with continuous intrathecal baclofen delivered via an implantable pump: long-term follow-up review of 18 patients. J Neurosurg 91 : 733-6.
Rex A, Schickert R, Fink H. Antidepressant-lile effect of nicotinamide adenine dinucleotide (NADH) in the forced swim test in rats. Pharmacol Biochem Behav 2004; in press
Richter A, Loscher W (1999). Gabapentin decreases the severity of dystonia at low doses in a genetic animal model of paroxysmal dystonic choreoathetosis. Eur J Pharmacol 369: 335-8.
Richter A, Loscher W (2000). Paradoxical aggravation of paroxysmal dystonia during chronic treatment with phenobarbital in a genetic rodent model. Eur J Pharmacol 397: 343-50.
Rodriguez-Pallares J, Caruncho HJ, Lopez-Real A, Wojcik S, Guerra MJ, Labandeira-Garcia JL (2001). Rat brain cholinergic, dopaminergic, noradrenergic and serotonergic neurons express GABAA receptors derived from the alpha3 subunit. Receptors Channels 7: 471-478.
Sams-Dodd F (1998). Effects of diazepam, citalopram, methadone and naloxone on PCP-induced stereotyped behaviour and social isolation in the rat social interaction test. Neurosci Biobehav Rev 23: 287-93.
Sawa A, Snyder SH (2002). Schizophrenia: diverse approaches to a complex disease. Science 296: 692-695.
Siep E, Richter A, Loscher W, Speckmann EJ, Kohling R (2002). Sodium currents in striatal neurons from dystonic dt(sz) hamsters: altered response to lamotrigine. Neurobiol Dis 9: 258-68.
Stokes PE, Holtz A (1997). Fluoxetine tenth anniversary update: the progress continues. Clin Ther 19: 1135-1250.
Tao R, Auerbach SB (2000). Regulation of serotonin release by GABA and excitatory amino acids. J Psychopharmacol 14: 100-113.
Tauber M, Calame-Droz E, Prut L, Rudolph U, Crestani F (2003). alpha2-gamma-Aminobutyric acid (GABA)A receptors are the molecular substrates mediating precipitation of narcosis but not of sedation by the combined use of diazepam and alcohol in vivo. Eur J Neurosci 18: 2599-2604.
Taylor CP, Gee NS, Su TZ, Kocsis JD, Welty DF, Brown JP, Dooley DJ, Boden P, Singh L (1998). A summary of mechanistic hypotheses of gabapentin pharmacology. Epilepsy Res 29: 233-249.
Thyagarajan D (2001). Genetics of movement disorders: an abbreviated overview. Stereotact Funct Meurosurg 77 : 48-60.
Vercueil L, Krack P, Pollak P (2002). Results of deep brain stimulation for dystonia: a critical reappraisal. Mov Disord 17: S89-93.
Willner P. Animal models of depression: an overview. Pharmacol Ther 1990; 45: 425-455
Willner P. The validity of animal models of depression. Psychopharmacology 1984; 83: 1-16
Wisden, W., Herb, A., Wieland, H., Keinänen, K., Lüddens, H., and Seeburg, P. H. (1991). Cloning, pharmacological characteristics and expression pattern of the rat GABAA receptor α4 subunit. FEBS Lett 289, 227-230.
Ziprasidone (CP-88,059): A new antipsychotic with combined dopamine and serotonin receptor antagonist activity. J Pharmacol Exp Ther 275: 101-13.

## Claims

1. Use of an effective amount of compound 1-(4-methoxy)-4-piperidinoimidazolin-2-one or 1-(4-chlorophenyl)-4-piperidinoimidazolin-2-one for the manufacture of a medicament for treating or preventing central nervous system disorders, wherein the central nervous system disorder is a mood disorder or mood episode or an anxiety disorder or episode of anxiety by administering to a patient in need thereof.

2. Use according to claim 1, wherein 1-(4-chlorophenyl)-4-piperidinoimidazolin-2-one is used.

3. Use according to claims 1 or 2, wherein the compound is formulated for parenteral or oral administration.

4. Use according to any one of claims 1 to 3 wherein the compound is provided for administration in an amount of 1-100 mg/kg of the body weight of the patient.

5. Use according to anyone of claims 1 to 4 wherein the central nervous system disorder is a mood disorder or mood episode.

6. Use according to claim 5, wherein the mood disorder or mood episode is a major depressive disorder or episode, manic, mixed and hypomanic mood episodes, depressive episodes with atypical catatonic or melancholic features, depressive episodes with postpartum onset premenstrual dysphoric disorder, minor depressive disorder, post traumatic, acute stress disorder, obsessive-compulsive disorder and patients with eating disorders.

7. Use according to anyone of claims1 to 4, wherein the central nervous system disorder is an anxiety disorder or episode of anxiety.

8. Use according to claim 7, wherein the anxiety disorder or episode is chronic anxiety disorder, panic disorder, agoraphobia, specific phobia, social phobia and generalized anxiety disorder.

9. Pharmaceutical composition for use in a method for the treatment or prevention of central nervous system disorders wherein the central nervous system disorder is a mood disorder or mood episode or an anxiety disorder or episode of anxiety containing an effective amount of 1-(4-methoxy)-4-piperidinoimidazolin-2-one or 1-(4-chlorophenyl)-4-piperidinoimidazolin-2-one, practised on a patient in need thereof.

10. Pharmaceutical composition according to claim 9, wherein the compound is 1-(4-chlorophenyl)-4-piperidinoimidazolin-2-one.

11. Pharmaceutical composition according to anyone of claims 9 to 10 wherein it contains further excipients or auxiliaries.

12. Pharmaceutical composition according to any one of claims 9 to 11 for parenteral or oral administration.

13. Pharmaceutical composition according to claim 9, wherein it contains 1-100 mg/kg body weight of the patient of the compound.

14. Pharmaceutical composition according to anyone of claims 9 to 13, wherein the central nervous system disorder is a mood disorder or mood episode.

15. Pharmaceutical composition according to claim 14, wherein the mood disorder or mood episode is a major depressive disorder or episode, manic, mixed and hypomanic mood episodes, depressive episodes with atypical, catatonic or melancholic features, depressive episodes with postpartum onset premenstrual dysphoric disorder, minor depressive disorder, post traumatic, acute stress disorder, obsessive-compulsive disorder and patients with eating disorders.

16. Pharmaceutical composition according to anyone of claims 9 to 13, wherein the central nervous system disorder is an anxiety disorder or episode of anxiety.

17. Pharmaceutical composition according to claim 16, wherein the anxiety disorder or episode is chronic anxiety disorder, panic disorder, agoraphobia, specific phobia, social phobia and generalized anxiety disorder.
